(19) Europäisches Patentamt European Patent Office Office européen des brevets

(11) **EP 3 575 791 B1**

(12) # EUROPEAN PATENT SPECIFICATION

(45) Date of publication and mention
of the grant of the patent:
**10.01.2024 Bulletin 2024/02**

(21) Application number: **19177533.7**

(22) Date of filing: **30.05.2019**

(51) International Patent Classification (IPC):
**G01N 33/49** (2006.01)  **C12N 5/078** (2010.01)
**A61B 5/15** (2006.01)  **G01N 33/50** (2006.01)

(52) Cooperative Patent Classification (CPC):
**C12N 5/0634; A61B 5/15; G01N 33/49;
G01N 33/491; G01N 33/5094;** C12N 2501/01

(54) **BLOOD TREATMENT METHOD AND BLOOD COLLECTION TUBE**

BLUTBEHANDLUNGSVERFAHREN UND BLUTENTNAHMERÖHRCHEN

PROCÉDÉ DE TRAITEMENT DU SANG ET TUBE DE COLLECTE DE SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2018 JP 2018104309**

(43) Date of publication of application:
**04.12.2019 Bulletin 2019/49**

(73) Proprietor: **ARKRAY, Inc.
Kyoto-shi, Kyoto 601-8045 (JP)**

(72) Inventor: **TAKAGI, Hidenori
Kyoto, 602-0008 (JP)**

(74) Representative: **Dehns
St. Bride's House
10 Salisbury Square
London EC4Y 8JD (GB)**

(56) References cited:
EP-A1- 2 848 935        EP-A1- 3 306 315
WO-A1-02/25280        WO-A1-03/090794
WO-A1-2013/116702    WO-A1-2015/113988
CN-B- 104 381 245      US-A- 4 994 367
US-A1- 2005 181 353    US-A1- 2011 027 771
US-A1- 2017 072 396

- **CONTANT G ET AL: "Heparin inactivation during blood storage : Its prevention by blood collection in citric acid, theophylline, adenosine, dipyridamole -C.T.A.D. mixture-", THROMBOSIS RESEARCH, TARRYTOWN, NY, US, vol. 31, no. 2, 15 July 1983 (1983-07-15), pages 365-374, XP022877896, ISSN: 0049-3848, DOI: 10.1016/0049-3848(83)90337-7 [retrieved on 1983-07-15]**

**Description**

BACKGROUND OF THE INVENTION

1. Field of the Invention

The present disclosure relates to a test for rare cells in blood, and a blood treatment method and a blood collection tube for the test.

2. Description of Related Art

Whole blood is one of the most frequently collected samples for diagnostic analysis in the medical field.

If whole blood is left untreated after being collected, undesired coagulation, metabolism, degradation, and the like of erythrocytes, leukocytes, platelets, and plasma components occur as time passes. Accordingly, it is desirable to perform a test or an analysis using whole blood immediately after blood is collected, however, in reality, this is often difficult, and transportation or storage of the whole blood may be required. To address this issue, an anticoagulant agent or the like is added to the collected blood in order to store the blood stably. There are various types of anticoagulant agents. In order to keep the tests from being affected, blood collection tubes for use in a method for treating collected blood and in blood collection are classified into various types according to the purpose of the tests.

For example, the blood collection tubes are largely classified into those not containing an anticoagulant agent (plain type) and those containing an anticoagulant agent. Blood collection tubes of the plain type are used in biochemical tests, endocrine tests, infectious disease tests, autoantibody tests, tumor marker tests, and the like. On the other hand, there are several types of blood collection tubes containing an anticoagulant agent, and a blood collection tube containing an anticoagulant agent that is suitable for the purpose of the test is used.

Blood collection tubes containing EDTA are used in hematology tests such as blood cell counts and blood smear tests.

Blood collection tubes containing sodium citrate or blood collection tubes containing CTAD are used in coagulation system tests. CTAD refers to a drug that contains a combination of citrate, theophylline, adenosine, and dipyridamole.

Blood collection tubes containing sodium fluoride (NaF) are used in blood sugar tests.

Blood collection tubes containing heparin are used in tests such as an electrolyte test, a blood pH test, a chromosomal analysis, and lymphocyte culture.

As blood collection tubes for use in a test for rare cells in blood, blood collection tubes containing EDTA (EDTA-2K or EDTA-3K) are commercially available.

The main solid components of blood are erythrocytes, leukocytes, and platelets. However, blood may also contain cells called "rare cells". Of the solid components in human peripheral blood, erythrocytes are present in the form of anucleate cells that lack mitochondria, a ribosome, a Golgi body, and an endoplasmic reticulum. The erythrocytes can be hemolyzed by diluting whole blood and adding ammonium chloride thereto. The leukocytes have a nucleus, and respond to proteins such as various chemokines and interferons. The platelets are anucleate cells, and are fragments of cytoplasms that are derived from the megakaryocytes. The platelets are activated by ADP. As described above, the components in whole blood greatly differ from each other in their skeletons, contents, and structures, cause distinctive reactions characteristic of them, and exhibit complex behaviors. Examples of the rare cells include circulating tumor cells (CTCs) and immunocytes. The rare cells are regarded as medically important cells. The CTCs are cancer cells (tumor cells) that are released from primary tumor tissues or metastatic tumor tissues and circulate through the bloodstream. It is said that metastases occur when the CTCs are carried to other organs and the like by this circulation through the bloodstream. On this account, CTCs in blood are recognized as a useful factor for determining the therapeutic effect against metastatic cancers and predicting prognosis, and thus CTC analysis is actively performed.

JP 2017-507328A discloses that, in an experimental system in which cancer cells are detected after adding the cancer cells to whole blood and then storing the whole blood at ordinary temperature for 48 hours, EDTA or citrate is used as an anticoagulant agent in order to improve the detection efficiency. This literature also discloses that CPDA (citrate, phosphate, glucose, and adenine) can be used as a blood stabilizer. This literature further discloses that not only EDTA, citrate, and CPDA but also Sivelestat (neutrophil elastase inhibitor), Pefabloc™ SC (serine protease inhibitor), and antioxidant agents such as glutathione, SkQ1, and MitoQ also improve the detection efficiency.

WO 2013/168767 and JP 2005-501236Apropose that, when blood to be used in a CTC test needs to be stored for a long time, the blood should be subjected to formaldehyde fixation.

SUMMARY OF THE INVENTION

10 ml of whole blood contains several tens of billions of erythrocytes, several tens of millions of leukocytes, and several billion platelets. In contrast, only several to several thousands CTCs are present in 10 ml of whole blood. When rare cells are to be detected after storing whole blood, not only the state of the rare cells but also the states of the erythrocytes, the leukocytes, and the platelets may affect the test result. For example, if aggregation occurs only in 1 vol% of 10 ml of blood (10 μl in volume), for example, about thirty million to sixty million erythrocytes, about thirty thousand to one hundred thousand leukocytes, and about one million to thirty million platelets and plasma components are involved in the aggregation. In rare cell tests using whole blood, aggregates of these components may degrade the treatment performance.

In particular, in the case of CTC tests, the use of whole blood that contains EDTA2K as an anticoagulant agent and has been stored for 1 to 2 days may adversely affect the detection result and the recovery rate of CTCs.

For example, in a separation method utilizing an antigen or in separation using a density-gradient method, erythrocytes and leukocytes may change over time during storage, thereby causing a change in density. Also, some immunocytes may die over time during storage, which leads to issues such as leakage of the cellular contents and aggregation of nucleic acids. In separation utilizing size or viscoelasticity, as a result of the change in blood over time during storage, the pressure difference at a separation portion increases as compared with the case where, for example, fresh blood is treated at the same flow rate. This causes a change in the passability of some CTCs such as cancer cells with high deformability and small-sized cancer cells through a filter (passability through small flow paths) and the like.

Furthermore, not only the state of the blood but also the state of the CTCs themselves may change over time during storage. Owing to cell death or degradation down of DNA and RNA, it may not be possible to successfully perform a test using blood that has been stored despite being able to perform the test when fresh blood is used. Some methods involve formaldehyde fixation of CTCs, which, however, may adversely affect the separation or may limit the use of the test.

On the other hand, when a test is to be performed in a facility (e.g., a testing organization) other than a hospital or the like where the blood collection is performed, it is necessary to store the collected blood.

With the foregoing in mind, the present disclosure provides a method for treating collected blood to be used in a test for rare cells in blood.

In particular, one aspect of the present disclosure relates to a method for treating collected blood to be used in a test for rare cells in blood, including: mixing the collected blood with cAMP or an analogous compound thereof selected from the group consisting of cAMP analogs, cGMP and cGMP analogs.

The present disclosure also relates to a method for performing a test for rare cells in blood, including: using blood treated using the method according to the present disclosure as a blood sample to be subjected to the test. The present disclosure also relates to a method for performing a test for rare cells in blood, comprising treating collected blood by a method according to the present disclosure and subjecting the treated collected blood (which is used as the sample) to a test for rare cells.

Still another aspect of the present disclosure relates to a blood collection tube for use in a test for rare cells in blood, containing: cAMP or an analogous compound thereof selected from the group consisting of cAMP analogs, cGMP and cGMP analogs. In an alternative the present disclosure provides use of a blood collection tube in a test for rare cells in blood, wherein the blood collection tube is as defined in the present disclosure.

Still another aspect of the present disclosure relates to a method for treating collected blood, including: mixing the collected blood with cAMP or an analogous compound thereof and with a cyclooxygenase (COX) inhibitor, or including: mixing the collected blood with cAMP and an analogous compound thereof, wherein the analogous compound is selected from the group consisting of cAMP analogs, cGMP and cGMP analogs.

Still another aspect of the present disclosure relates to a blood collection tube containing: cAMP or an analogous compound thereof and a cyclooxygenase (COX) inhibitor, or containing: cAMP or an analogous compound thereof, wherein the analogous compound is selected from the group consisting of cAMP analogs, cGMP and cGMP analogs.

According to the present disclosure, in one or more embodiments, a rare cell test using blood that has been stored after being collected can be performed more efficiently.

Also, according to the present disclosure, in one or more embodiments, a rare cell test using blood that has been stored after being collected can be performed more efficiently regardless of whether or not cell immobilization is performed.

BRIEF DESCRIPTION OF THE DRAWINGS

FIG. 1 is a graph showing an example of the results (capture rates) of a CTC test performed on samples prepared by mixing collected blood and additives of Examples 9 to 18 and Comparative Example 8, respectively, and then storing the mixtures at 4°C for 72 hours.

FIGs. 2A and 2B show an example of the results of DNA-FISH (on the left) and RNA-FISH (on the right) of CTCs collected from samples prepared using the method for treating collected blood according to the present disclosure, and FIG. 2B is an image schematically showing fluorescent staining in FIG. 2A.

DETAILED DESCRIPTION OF THE INVENTION

The present disclosure is based on the finding that, by adding an agent that increases the intracellular concentration of cAMP or an analogous compound thereof to blood to be used in a rare cell test when blood is to be stored, the efficiency of the rare cell test can be improved.

Rare Cells

In the present disclosure, "rare cells" that may be contained in blood or a blood specimen refer to cells other than cell components (erythrocytes, leukocytes, and platelets) that may be contained in the blood of a human or an animal other than a human, and encompass tumor cells and/or cancer cells. A tumor cell or a cancer cell that circulates in the blood is generally called a circulating tumor cell (CTC). The number of rare cells in blood varies depending on the specimen, and 10 ml of blood may contain several to several tens of rare cells, and several hundreds to several thousands of rare cells at most. In one or more embodiments, the "rare cells" are cells selected from the group consisting of cancer cells, circulating tumor cells, vascular endothelial cells, vascular endothelial precursor cells, cancer stem cells, epithelial cells, hematopoietic stem cells, mesenchymal stem cells, fetal cells, stem cells, and combinations thereof.

Rare Cell Test

In the present disclosure, a rare cell test may encompass rare cell detection, rare cell separation, rare cell enrichment, rare cell measurement, rare cell observation, rare cell recovery, rare cell culture, and tests for DNA, RNA, a protein, or the like contained in rare cells.

The method for performing the rare cell test is not particularly limited. Examples of the method include: methods using size and viscoelasticity, such as separation by filtration; methods using size difference and viscoelasticity, such as separation using flow paths and hydrodynamic separation; methods using centrifugation; methods involving an immunological technique such as the use of an antibody; methods using staining; methods using a microscope, and combinations thereof.

Examples of the improvement in the efficiency of the rare cell test include improvement in test accuracy, improvement in detection sensitivity, improvement in separation accuracy, improvement in the degree of enrichment, improvement in measurement accuracy, improvement in ease of observation, improvement in recovery rate, and improvement in survival rate of rare cells.

In the present disclosure, the rare cell test may involve immobilizing cells through formalin fixation or the like, or may be performed without immobilizing the cells. From the viewpoint of subjecting living rare cells to observation, measurement, and/or cultivation, it is preferable to perform the rare cell test without immobilizing the cells.

Blood

In the present disclosure, blood may be blood of a human or an animal other than a human. When the treatment method according to the present disclosure is applied to blood containing leukocyte components, an effect obtained by stabilizing leukocytes and/or other nucleated cells can be more prominent.

Agent that Increases Intracellular Concentration of cAMP or Analogous Compound Thereof

In general terms, the present disclosure is concerned with methods for treating collected blood to be used in a test for rare cells in blood, including: mixing the collected blood with an agent that increases the intracellular concentration of cAMP or an analogous compound thereof. In this regard, examples of the agent that increases the intracellular concentration of cAMP (cyclic adenosine monophosphate, cyclic AMP) or an analogous compound thereof include cAMP and analogous compounds thereof, substances that suppress intracellular degradation of cAMP, and substances that promote intracellular synthesis of cAMP. However, aspects of the invention are directed to the use of cAMP or an analogous compound thereof selected from cAMP analogs, cGMP and cGMP analogs.

cAMP and Analogous Compounds Thereof

cAMP is a cyclic nucleotide, and is known as an intracellular transmitter (second messenger). For example, it is known that cAMP activates cAMP-dependent protein kinase (PKA) and regulates various cell responses such as specific gene expression of nucleated cells. cAMP exhibits various effects and functions depending on the cell.

In accordance with the present invention, the analogous compounds of cAMP are cAMP analogs, cGMP (cyclic guanosine monophosphate, cyclic GMP), and cGMP analogs.

In the present disclosure, the cAMP analogs refer to substances that have a structure similar to that of cAMP and that respectively supply cAMP after being degraded in cells or have functions equivalent to those of cAMP as they are.

Similarly to cAMP, cGMP is also a cyclic nucleotide and is known as a second messenger.

In the present disclosure, cGMP analogs refer to substances having a structure similar to that of cGMP and that respectively supply cGMP after being degraded in cells or have functions equivalent to those of cGMP as they are.

As the cAMP analogs and the cGMP analogs, the following substances are more preferable: substances that can be introduced into cells; substances that are easily taken up into cells; or substances that can easily pass through cell membranes.

Examples of the cAMP analog include 8-bromo-cAMP sodium salt (8-Br-cAMP, CAS No. 76939-46-3), 6-monobutyryl cAMP (6MB-cAMP, CAS No. 70253-67-7), dibutyryl cAMP sodium salt (DBcAMP, CAS No. 16980-89-5), 8-(4-chlorophenylthio)-cAMP sodium salt (8-pCPTcAMP, CAS No. 93882-12-3), 8-(4-chlorophenylthio)-2'-O-methyl cAMP sodium salt (8-CPT-2'-O-Me-cAMP, CAS No. 510774-50-2), 2-chloro-8 methylamino cAMP (2-Cl-8-MA-cAMP, CAS No. 96990-16-8), N-[6-(N-methylanthraniloylamino)hexyl]-cAMP (6-MAH-cAMP, CAS No. 723313-27-7), and 8-(6-aminohexyl)amino-cAMP: 8-AHA-cAMP (CAS No. 39824-30-1).

Examples of the cGMP analog include 8-bromo-cGMP (8-Br-GMP, CAS No. 51116-01-9), dibutyryl-cGMP (DBcAMP, CAS No. 51116-00-8), and 8-(4-chlorophenylthio)-guanosine 3',5'-cyclic monophosphate sodium salt (8-pCPT-cGMP, CAS No. 51239-26-0).

Out of cAMP and the analogous compounds thereof, the cAMP analogs and the cGMP analogs are preferable, and the cAMP analogs are more preferable, from the viewpoint of ease of passing through cell membranes and improving the efficiency of a rare cell test.

When any of cAMP and the analogous compounds thereof, in particular, cAMP or cGMP, is used as the agent that increases the intracellular concentration of cAMP or an analogous compound thereof, a reagent for introducing cAMP and the analogous compounds thereof into cells may be used in combination, if necessary. For example, it is possible to make the passage of cAMP and cGMP through cell membranes easier by encapsulating them in a lipid such as a liposome, perforating the cell membranes using a surfactant, using a solvent, or utilizing a reagent that promotes endocytosis. Such a reagent may be contained in the agent that increases the intracellular concentration of cAMP or an analogous compound thereof, or may be mixed with the blood separately from the agent.

When cAMP or an analogous compound thereof is mixed with the blood, the final concentration thereof may be, for example, from 1 pM to 1M and preferably from 1 nM to 100 mM.

From the viewpoint of improving the efficiency of a rare cell test, the present disclosure may include using an agent (introduction promoter) that promotes introduction of the agent that increases the intracellular concentration of cAMP or an analogous compound thereof into cells in combination. Examples of the introduction promoter include reagents for introducing cAMP and analogous compounds thereof to cells, surfactants, solvents, and reagents that promote endocytosis.

Substances that Suppress Intracellular Degradation of cAMP

cAMP is degraded in cells by an enzyme called phosphodiesterase (PDE). By inhibiting the phosphodiesterase, cAMP accumulates in cells without being degraded, and the amount of cAMP in the cells can thus be increased more easily. Accordingly, a substance that suppresses intracellular degradation of cAMP may be a phosphodiesterase inhibitor.

Examples of the phosphodiesterase inhibitor include xanthines (xanthine derivatives), inhibitors against PDE3 and/or PDE5 in platelets that are a blood component, and inhibitors against PDE4, PDE7, and/or PDE8 in immunocytes.

The phosphodiesterase inhibitor is preferably a phosphodiesterase inhibitor that can inhibit phosphodiesterases in platelets and/or immunocytes from the viewpoint of improving the efficiency of a rare cell test. Examples of such a phosphodiesterase inhibitor include inhibitor against PDE3 in platelets and inhibitor against PDE5 in platelets and inhibitor against PDE4 in immunocytes, inhibitor against PDE7 in immunocytes, and inhibitor against PDE8 in immunocytes. From the viewpoint of improving the efficiency of a rare cell test, the phosphodiesterase inhibitor may contain at least substances that inhibit the phosphodiesterases in immunocytes, and may contain at least substances that specifically inhibit the phosphodiesterases in immunocytes. The phosphodiesterase inhibitor may also contain substances that inhibit the phosphodiesterases in platelets and substances that specifically inhibit the phosphodiesterases in immunocytes.

Examples of a xanthine phosphodiesterase inhibitor include PDE inhibitors with low specificity, such as aminophylline (CAS No. 317-34-0), caffeine (CAS No. 58-08-2), xanthine (CAS No. 69-89-6), theophylline (CAS No. 58-55-9), choline theophylline (CAS No. 4499-40-5), theophylline-7-acetic acid (CAS No. 652-37-9), 8-bromotheophylline (CAS No. 10381-75-6), theobromine (CAS No. 83-67-0), diprophylline (CAS479-18-5), and etofylline (CAS No. 519-37-9).

Examples of the inhibitor specific to PDE3 include cilostazol (CAS No. 73963-72-1), milrinone (CAS No. 78415-72-2), amrinone (CAS No. 60719-84-8), pimobendan (CAS No. 74150-27-9), an anagrelide hydrochloride hydrate (CAS No. 823178-43-4), cilostamide (CAS No. 68550-75-4), enoximone (CAS No. 77671-31-9), and trequinsin hydrochloride (CAS No. 78416-81-6).

Examples of the inhibitor specific to PDE5 include zaprinast (CAS No. 37762-06-4), sildenafil (CAS No. 171599-83-0), vardenafil dihydrochloride (CAS No. 224789-15-5), tadalafil (CAS No. 171596-29-5), MY-5445 (CAS No. 78351-75-4), avanafil (CAS No. 330784-47-9), udenafil (CAS No. 268203-93-6), gisadenafil (CAS No. 334827-98-4), OSI-461 (CAS No. 227619-96-7), dipyridamole (CAS No. 58-32-2), and PDE5 inhibitor 42 (CAS No. 936449-28-4).

Examples of the inhibitor specific to PDE4 include rolipram (CAS No. 61413-54-5), roflumilast (CAS No. 162401-32-3), cilomilast (CAS No. 153259-65-5), Ro-20-1724 (CAS No. 29925-17-5), OPC6535 (CAS No. 145739-56-6), etazolate hydrochloride (CAS No. 35838-58-5), denbufylline (CAS No. 57076-71-8), doxofylline (CAS No. 69975-86-6), arofylline (CAS No. 136145-07-8), apremilast (CAS No. 608141-41-9), BAY 19-8004 (CAS No. 329306-27-6), and L 454560 (CAS No. 346629-30-9).

Examples of the inhibitor specific to PDE7 or PDE 8 include imidazopyridine or a derivative thereof, dihydropurine or a derivative thereof, pyrrole or a derivative thereof, benzothiopyranoimidazolone or a derivative thereof, heterocyclic compounds, quinazoline or a derivative thereof, pyridopyrimidine or a derivative thereof, tricyclic spiro derivatives, thiazole or a derivative thereof, oxathiazole or a derivative thereof, sulfonamide or a derivative thereof, heterobiaryl sulfonamide or a derivative thereof, dihydroisoquinoline or a derivative thereof, guanine or a derivative thereof, benzothiadiazine or a derivative thereof, and benzothienothiazine or a derivative thereof. More specific examples thereof include BRL 50481 (CAS433695-36-4), ASB-16165 (CAS No. 873541-45-8), and dipyridamole (CAS No. 58-32-2). Dipyridamole also inhibits PDE5, and thus also exhibits an effect of suppressing platelet aggregation.

A composition that contains, out of the above-described phosphodiesterase inhibitors, dipyridamole and theophylline is, for example, a composition (CTAD) containing the combination of citrate, theophylline, adenosine, and dipyridamole. Although blood collection tubes containing CTAD are commercially available, they are for use in coagulation system tests and not for use in rare cell tests.

When the substance that suppresses intracellular degradation of cAMP is mixed with the blood, the final concentration of the substance is, for example, from 1 pM to 100 mM and preferably from 1 nM to 1 mM in the case where the substance is, e.g., dipyridamole, and is, for example, from 1 pM to 1M and preferably from 1 $\mu$M to 100 mM in the case where the substance is theophylline.

Substances that Promote Intracellular Synthesis of cAMP

In cells, cAMP and cGMP are synthesized from ATP and GTP through adenylate cyclase and guanylate cyclase, respectively. Accordingly, as a substance that promotes intracellular synthesis of cAMP, a substance may be used that promotes intracellular synthesis of cAMP by activating and/or maintaining adenylate cyclase or guanylate cyclase through receptors that are necessary to enhance the degradation of cAMP from ATP (e.g., adenosine receptors and prostacyclin receptors) or through inhibition of ADP receptors. The substance that promotes intracellular synthesis of cAMP in such a manner may be an activator that increases cAMP in platelets and immunocytes. Examples of such an activator include adenosine (CAS No. 58-61-7), 2-chloroadenosine (CAS No. 146-77-0), beraprost sodium (CAS No. 88475-69-8), prostacyclin (CAS No. 35121-78-9), ticlopidine (CAS No. 55142-85-3), clopidogrel (CAS No. 113665-84-2), forskolin (CAS No. 66575-29-9), and NKH 447 (CAS No. 138605-00-2).

CTAD, which contains theophylline and dipyridamole (phosphodiesterase inhibitors) in addition to adenosine, can be used as a combination of the substance that promotes intracellular synthesis of cAMP and the substance that suppresses intracellular degradation of cAMP, i.e., as a combination of the agents that increase the intracellular concentration of cAMP or an analogous compound thereof.

When the substance that promotes intracellular synthesis of cAMP is mixed with the blood, the final concentration of the substance is, for example, from 1 pM to 1 M and preferably from 1 nM to 100 mM in the case where the substance is adenosine, for example.

One type of the agent that increases the intracellular concentration of cAMP or an analogous compound thereof may be used alone, or two or more types of the agents may be used in combination. For example, the combination of two or more types of the agents may be a combination of cAMP or an analogous compound thereof and a substance that suppresses intracellular degradation of cAMP, a combination of cAMP or an analogous compound thereof and a substance that promotes intracellular synthesis of cAMP, or a combination of a substance that suppresses intracellular degradation of cAMP and a substance that promotes intracellular synthesis of cAMP. Furthermore, the combination of two or more types of the agents may be a combination of cAMP or an analogous compound thereof, a substance that suppresses intracellular degradation of cAMP, and a substance that promotes intracellular synthesis of cAMP.

Specifically, such a combination may be a combination of cAMP or an analogous compound thereof and CTAD.

Method for Treating Collected Blood

The present disclosure provides a method for treating collected blood to be used in a test for rare cells in blood, including mixing the collected blood with an agent that increases the intracellular concentration of cAMP or an analogous compound thereof.

The "agent that increases the intracellular concentration of cAMP or an analogous compound thereof" is as described above. In methods of the invention the agent that increases the intracellular concentration of cAMP or an analogous compound thereof is cAMP or an analogous compound thereof selected from cAMP analogs, cGMP and cGMP analogs

In one or more examples, mixing of the collected blood with the agent that increases the intracellular concentration of cAMP or an analogous compound thereof can be performed by collecting blood using a blood collection tube that contains the agent that increases the intracellular concentration of cAMP or an analogous compound thereof. In one or more other examples, the agent that increases the intracellular concentration of cAMP or an analogous compound thereof may be added to blood after blood is collected. The blood to be mixed with the agent that increases the intracellular concentration of cAMP or an analogous compound thereof may already contain other components to be described below, such as an anticoagulant agent.

In the blood treated using the treatment method according to the present disclosure, platelets, leukocytes, and rare cells are stabilized. Accordingly, the storage stability of a rare cell test is improved, whereby the efficiency of the rare cell test can be improved. This advantageous effect can be exhibited even if cells are not immobilized during the rare cell test.

The storage period over which the blood treated with the treatment method according to the present disclosure is stored until a rare cell test is performed (the period from the completion of the treatment (mixing) to the start of the test) is 4 hours or more, 6 hours or more, 12 hours or more, from 1 to 7 days, from 1 to 5 days, or from 1 to 3 days.

The blood treated using the treatment method according to the present disclosure may be stored at ordinary temperature or at a low temperature until the rare cell test is performed. When the blood is stored at a low temperature, the blood may be brought to room temperature or may be heated to a temperature from 29°C to 40°C before the rare cell test is performed.

In the present disclosure, the ordinary temperature may be, for example, from 15°C to 28°C. In the present disclosure, the low temperature may be, for example, 10°C or less, from 1°C to 10°C, from 2°C to 8°C, from 2°C to 5°C, or 4°C. From the viewpoint of improving the efficiency of the rare cell test, storage at a low temperature is preferable.

In one or more embodiments, the blood may be stored while being stirred, while being subjected to inversion mixing, or while being kept standing still. In one or more embodiments, the storage may encompass transportation.

Anticoagulant Agent

In the treatment method according to the present disclosure, in one or more examples, it is preferable to mix an anticoagulant agent or a composition containing the anticoagulant agent with the blood from the viewpoint of improving the efficiency of a rare cell test.

By adding the anticoagulant agent, plasma components, platelets, and cells in the blood are stabilized, and it is considered that this contributes to improvement in the efficiency of a rare cell test, such as improvement in the capture rate of rare cells by a filter.

Therefore, in one or more examples, the present disclosure relates to a method for treating collected blood to be used in a test for rare cells in blood, including mixing the collected blood with an agent that increases the intracellular concentration of cAMP or an analogous compound thereof and an anticoagulant agent.

When the collected blood is mixed with multiple agents in the treatment method according to the present disclosure, the multiple agents may be mixed with the collected blood simultaneously or sequentially. For example, the agent that increases the intracellular concentration of cAMP or an analogous compound thereof may be added after adding the anticoagulant agent, or the anticoagulant agent may be added after adding the agent that increases the intracellular concentration of cAMP or an analogous compound thereof.

The anticoagulant agent to be used in the treatment method according to the present disclosure may be heparin, warfarin, or a chelating agent, and examples of the chelating agent include citric acid and EDTA. When an anticoagulant agent is to be added, it is desirable to use citric acid as the anticoagulant agent.

In the present disclosure, the term "citric acid" encompasses citrate. Also, in the present disclosure, EDTA may encompass EDTA-2K and EDTA-3K.

CTAD, which contains citric acid, theophylline, adenosine, and dipyridamole, can be used as the agent that increases the intracellular concentration of cAMP or an analogous compound thereof and the anticoagulant agent.

When the anticoagulant agent is mixed with the blood, the final concentration of the anticoagulant agent is, for example, from 1 nM to 1M and preferably from 1 mM to 100 mM in the case where the anticoagulant agent is citric acid, for example.

Cyclooxygenase (COX) Inhibitor

In the treatment method according to the present disclosure, in one or more examples, it is preferable to mix a COX inhibitor or a composition containing the COX inhibitor with the blood from the viewpoint of improving the efficiency of a rare cell test in the case where the treated blood is stored at a low temperature.

By adding the COX inhibitor, aggregation during storage of the blood at low temperatures and inflammatory reactions in the blood are suppressed, although the details of the mechanism thereof have not been clarified. It is considered that this contributes to an improvement in the efficiency of a rare cell test, such as improvement in the capture rate of rare cells by a filter.

Therefore, in one or more examples, the present disclosure relates to a method for treating collected blood to be used in a test for rare cells in blood, including mixing the collected blood with an agent that increases the intracellular concentration of cAMP or an analogous compound thereof and a COX inhibitor.

In one or more other examples, the present disclosure relates to a method for treating collected blood to be used in a test for rare cells in blood, including mixing the collected blood with an agent that increases the intracellular concentration of cAMP or an analogous compound thereof, an anticoagulant agent, and a COX inhibitor.

The COX inhibitor to be used in the treatment method according to the present disclosure is, for example, a nonsteroidal anti-inflammatory drug (NSAID). Examples of NSAID include salicylic acid compounds, arylacetic acid derivatives, pyranoacetic acid compounds, propionic acid compounds, indoleacetic acid compounds, oxicam compounds, and arylacetic acid compounds. Specific examples of the COX inhibitor include sodium salicylate (CAS No. 54-21-7), aspirin (CAS No. 493-53-8), diclofenac sodium (CAS No. 15307-79-6), etodolac (CAS No. 41340-25-4), ibuprofen (CAS No. 15687-27-1), a loxoprofen sodium salt dihydrate (CAS No. 226721-96-6), oxaprozin (CAS No. 21256-18-8), naproxen (CAS No. 22204-53-1), meloxicam (CAS No. 71125-38-7), piroxicam (CAS No. 36322-90-4), indomethacin (CAS No. 53-86-1), SC560 (CAS No. 188817-13-2), sulindac (CAS No. 38194-50-2), resveratrol (CAS No. 501-36-0), niflumic acid (CAS No. 4394-00-7), NS-398 (CAS No. 123653-11-2), nimesulide (51803-78-2), and meclofenamic acid sodium salt (CAS No. 6385-02-0).

When the COX inhibitor is mixed with the blood, the final concentration of the COX inhibitor is, for example, from 1 pM to 100 mM and preferably from 10 nM to 10 mM in the case where the COX inhibitor is aspirin, for example.

Glucose

In the treatment method according to the present disclosure, in one or more examples, it is preferable to mix glucose or a composition containing glucose with the blood from the viewpoint of improving the efficiency of a rare cell test.

By adding glucose, ATP is synthesized from glucose in cells, although the details of the mechanism thereof have not been clarified. It is considered that cAMP can be increased owing to the increased supply of ATP, which is a substrate of adenylate cyclase (cAMP synthase). Furthermore, the deformability and/or the shape of erythrocytes are stabilized and the cell viability is also improved. It is considered that this contributes to an improvement in the efficiency of a rare cell test, such as an improvement in the recovery rate and capture rate of rare cells using density gradient separation, separation utilizing flow paths, or separation by a filter. In order to obtain an improved effect, citrate, phosphoric acid, adenine, inosine, mannitol, fructose, or the like further may be added.

Therefore, in one or more examples, the present disclosure relates to a method for treating collected blood to be used in a test for rare cells in blood, including mixing the collected blood with an agent that increases the intracellular concentration of cAMP or an analogous compound thereof and glucose.

In one or more other examples, the present disclosure relates to a method for treating collected blood to be used in a test for rare cells in blood, including mixing the collected blood with an agent that increases the intracellular concentration of cAMP or an analogous compound thereof, an anticoagulant agent, and glucose.

In one or more still other examples, the present disclosure relates to a method for treating collected blood to be used in a test for rare cells in blood, including mixing the collected blood with an agent that increases the intracellular concentration of cAMP or an analogous compound thereof, an anticoagulant agent, a COX inhibitor, and glucose.

Examples of the composition containing glucose include: a composition (CPDA) containing the combination of citrate, phosphate, glucose, and adenine; a composition (MDA) containing the combination of mannitol, glucose, and adenine; and a composition (MDAI) containing the combination of mannitol, glucose, adenine, and inosine.

CPDA, which contains citric acid and glucose, can be used as the anticoagulant agent and the glucose.

When the glucose is mixed with the blood, the final concentration of the glucose is, for example, from 1 nM to 1M and preferably from 1 $\mu$M to 400 mM.

Granulocyte Elastase Inhibitor

In the treatment method according to the present disclosure, in one or more examples, it is preferable to mix a granulocyte elastase inhibitor or a composition containing the granulocyte elastase inhibitor with the blood from the viewpoint of improving the efficiency of a rare cell test.

By adding the granulocyte elastase inhibitor, damage to cells and aggregation of the cells are suppressed by the inhibition of granulocyte elastase, although the details of the mechanism thereof have not been clarified. It is considered that this contributes to an improvement in the efficiency of a rare cell test, such as an improvement in the capture rate of rare cells by a filter.

Therefore, in one or more examples, the present disclosure relates to a method for treating collected blood to be used in a test for rare cells in blood, including mixing the collected blood with an agent that increases the intracellular concentration of cAMP or an analogous compound thereof and a granulocyte elastase inhibitor.

In one or more other examples, the present disclosure relates to a method for treating collected blood to be used in a test for rare cells in blood, including mixing the collected blood with an agent that increases the intracellular concentration of cAMP or an analogous compound thereof, an anticoagulant agent, and a granulocyte elastase inhibitor.

In one or more still other examples, the present disclosure relates to a method for treating collected blood to be used in a test for rare cells in blood, including mixing the collected blood with an agent that increases the intracellular concentration of cAMP or an analogous compound thereof, an anticoagulant agent, a COX inhibitor, and a granulocyte elastase inhibitor.

In one or more still other examples, the present disclosure relates to a method for treating collected blood to be used in a test for rare cells in blood, including mixing the collected blood with an agent that increases the intracellular concentration of cAMP or an analogous compound thereof, an anticoagulant agent, a COX inhibitor, glucose, and a granulocyte elastase inhibitor.

Examples of the granulocyte elastase inhibitor include sivelestat (CAS No. 127373-66-4), DMP-777 (CAS No. 157341-41-8), SSR 69071 (CAS No. 344930-95-6), elastatinal (CAS No. 51798-45-9), N-(methoxysuccinyl)-Ala-Ala-Pro-Val-chloromethyl ketone (CAS No. 65144-34-5), keracyanin chloride (CAS No. 18719-76-1), a trypsin inhibitor (derived from soybeans) (CAS No. 9035-81-8), 3,4-dichloroisocoumarin (CAS No. 51050-59-0), GW 311616 (CAS No. 198062-54-3), BAY678 (CAS No. 675103-36-3), 1-(3-methylbenzoyl)-1H-indazole-3-carbonitril (CAS No. 1448314-31-5), MR-889 (94149-41-4), CE-1037 (150493-09-7), andAE-3763 (CAS No. 291778-77-3).

When the granulocyte elastase inhibitor is mixed with the blood, the final concentration of the granulocyte elastase inhibitor is, for example, from 1 pM to 1M and preferably from 10 pM to 10 mM, in the case where the granulocyte elastase inhibitor is sivelestat, for example.

Antioxidant Agent

In the treatment method according to the present disclosure, in one or more examples, it is preferable to mix an antioxidant agent or a composition containing an antioxidant agent with the blood from the viewpoint of improving the efficiency of a rare cell test.

By adding the antioxidant agent, the cell viability is improved, although the details of the mechanism thereof have not been clarified. It is considered that this contributes to an improvement in the efficiency of a rare cell test, such as an improvement in the capture rate of rare cells by a filter.

Therefore, in one or more examples, the present disclosure relates to a method for treating collected blood to be used in a test for rare cells in blood, including mixing the collected blood with an agent that increases the intracellular concentration of cAMP or an analogous compound thereof and an antioxidant agent.

In one or more other examples, the present disclosure relates to a method for treating collected blood to be used in a test for rare cells in blood, including mixing the collected blood with an agent that increases the intracellular concentration of cAMP or an analogous compound thereof, an anticoagulant agent, and an antioxidant agent.

In one or more still other examples, the present disclosure relates to a method for treating collected blood to be used in a test for rare cells in blood, including mixing the collected blood with an agent that increases the intracellular concentration of cAMP or an analogous compound thereof, an anticoagulant agent, a COX inhibitor, and an antioxidant agent.

In one or more still other examples, the present disclosure relates to a method for treating collected blood to be used in a test for rare cells in blood, including mixing the collected blood with an agent that increases the intracellular concentration of cAMP or an analogous compound thereof, an anticoagulant agent, a COX inhibitor, glucose, and an antioxidant agent.

In one or more still other examples, the present disclosure relates to a method for treating collected blood to be used in a test for rare cells in blood, including mixing the collected blood with an agent that increases the intracellular concentration of cAMP or an analogous compound thereof, an anticoagulant agent, a COX inhibitor, glucose, a granulocyte elastase inhibitor, and an antioxidant agent.

Examples of the antioxidant agent include ascorbic acid, ascorbic acid 2-glucoside, ascorbyl tetra-2-hexyldecanoate, trisodium ascorbyl palmitate phosphate, melatonin, and caffeic acid.

The antioxidant agent may be an antioxidant agent targeted to mitochondria. Specific examples of such an antioxidant agent include SkQ1, MitoQ, and SS-31.

SkQ1 is known as a salt containing 10-(6'-plastoquinonyl)decyltriphenylphosphonium. In particular, SkQ 1 is known for its neuroprotective properties. It has been revealed that SkQ 1 inhibits oxidative stress caused by reactive oxygen species in mitochondria.

MitoQ is a mixture of mitoquinol (reduced form) and mitoquinone (oxidized form), which are also known as [10-(2,5-dihydroxy-3,4-dimethoxy-6-methylphenyl)decyl]triphenylphosphonium, and [10-(4,5-dimethoxy-2-methyl-3,6-dioxocyclohexa-1,4-dienyl)decylhriphenylphosphonium. MitoQ includes a ubiquinol moiety, and this moiety covalently binds to a lipophilic triphenylphosphonium cation via an aliphatic carbon chain. Antioxidant reactions of MitoQ mainly occur in a phospholipid bilayer.

SS-31 (d-Arg-Dmt-Lys-Phe-NH2; Dmt=2',6'-dimethyltyrosine) is a member of a family including several cell-permeable antioxidant peptides that reduce intracellular free radicals. It is known that, similarly to other members of the family, SS-31 peptide is targeted to mitochondria, and protects mitochondria from mitochondria permeability transition, swelling, and cytochrome c release and also prevents t-butylhydroperoxide-induced apoptosis.

When the antioxidant agent is mixed with the blood, the final concentration of the antioxidant agent is, for example, from 1 pM to 1M and preferably from 10 pM to 10 mM.

Other Components

In the treatment method according to the present disclosure, in one or more examples, the blood may be mixed with one or more components other than the above-described components as long as the other components do not greatly interfere with the rare cell test. Example of the other components include antibody reagents, enzyme reagents, surfactants, dye reagents, polymeric reagents, chemical reagents, inhibitory reagents, DNA and/or RNA probes, buffer solutions, diluents, and particles.

Method for Performing Rare Cell Test

The present disclosure relates to a method for performing a rare cell test using blood treated using the treatment method according to the present disclosure. In an alternative, the present disclosure relates to a method for performing a rare cell test (or for performing a test for rare cells) in blood, in which the method comprises treating collected blood using the treatment method according to the present disclosure and subjecting the collected blood to a rare cell test (or test for rare cells). The rare cell test is as described in the disclosure. These methods form aspects of the invention when using methods for treating blood according to the invention.

The method for performing the rare cell test is not particularly limited. Examples of the method include: methods using size and viscoelasticity, such as separation by filtration; methods using size difference and viscoelasticity, such as separation using flow paths and hydrodynamic separation; methods using centrifugation; methods involving an immunological technique such as the use of an antibody; methods using staining; methods using a microscope, and combinations thereof.

The rare cell test method may include storing a blood sample treated using the treatment method according to the present disclosure until the rare cell test is performed.

In one or more examples, the storage period over which the blood treated with the treatment method according to the present disclosure is stored until the rare cell test is performed is 4 hours or more, 6 hours or more, 12 hours or more, from 1 to 7 days, from 1 to 5 days, or from 1 to 3 days.

In one or more embodiments, the blood may be stored at ordinary temperature or at a low temperature. From the viewpoint of improving the efficiency of the rare cell test, storage at a low temperature is preferable. After storage, the blood may be heated before the rare cell test is performed, may be used after being brought to ordinary temperature, or may be treated after being heated to a temperature from 29°C to 40°C.

In the present disclosure, the ordinary temperature may be, for example, from 15°C to 28°C. In the present disclosure, the low temperature may be, for example, 10°C or less, from 1°C to 10°C, from 2°C to 8°C, from 2°C to 5°C, or 4°C.

In one or more embodiments, the storage may be performed while keeping the blood standing still and/or by transporting the blood.

Method for Treating Collected Blood (Second Aspect)

The treatment method according to the present disclosure may be used in blood component tests other than the rare cell test. This is because the treatment method according to the present disclosure stabilizes blood components. Therefore, another example of the present disclosure relates to a method for treating collected blood (second aspect), including mixing the collected blood with an agent that increases the intracellular concentration of cAMP or an analogous compound thereof.

As to the agent that increases the intracellular concentration of cAMP or an analogous compound thereof and other agents (anticoagulant agent, COX inhibitor, glucose, granulocyte elastase inhibitor, antioxidant agent, etc.) to be mixed with the blood, reference can be made to the descriptions regarding these agents in the above-described treatment method according to the present disclosure.

In one or more examples, the method for treating collected blood according to the present disclosure (second aspect) may be a method including mixing the collected blood with an agent that increases the intracellular concentration of cAMP or an analogous compound thereof and a COX inhibitor.

In one or more other examples, the method for treating collected blood according to the present disclosure (second aspect) may be a method including mixing the collected blood with cAMP or an analogous compound thereof.

Method for Performing Blood Component Test

The present disclosure also relates to a method for performing a blood component test, including performing a blood component test using blood treated using the treatment method according to the present disclosure (second aspect).

The method for performing the blood component test is not particularly limited, and a method commonly performed depending on the purpose of the test may be employed.

Blood Collection Tube

The present disclosure also relates to a blood collection tube for use in a test for rare cells in blood, containing an agent that increases the intracellular concentration of cAMP or an analogous compound thereof. In aspects according to the invention the agent is cAMP or an analogous compound thereof selected from cAMP analogs, cGMP and cGMP analogs.

In the present disclosure, the state where the blood collection tube "contains an agent" may mean that the agent is present in the blood collection tube in such a manner that blood that has been collected and is to be introduced into the blood collection tube can be brought into contact with the agent.

The technical feature of the blood collection tube according to the present disclosure is the combination of the use thereof in a rare cell test and the agent contained in the blood collection tube, and there is no particular limitation on the material, size, and the like of the blood collection tube.

The agent that increases the intracellular concentration of cAMP or an analogous compound thereof to be contained in the blood collection tube according to the present disclosure may be cAMP or an analogous compound thereof, a substance that suppresses intracellular degradation of cAMP, or a substance that promotes intracellular synthesis of cAMP. The respective substances are as described above. The amount of the agent that increases the intracellular concentration of cAMP or an analogous compound thereof to be contained in the blood collection tube may be such that the above-described final concentration of the agent is achieved when a predetermined amount of collected blood is introduced into the blood collection tube. In aspects according to the invention the agent is cAMP or an analogous compound thereof selected from cAMP analogs, cGMP and cGMP analogs.

The blood collection tube according to the present disclosure may contain one type of the agent that increases the intracellular concentration of cAMP or an analogous compound thereof alone, or may contain two or more types of the agents in combination. For example, the combination of two or more types of the agents may be a combination of cAMP or an analogous compound thereof and a substance that suppresses intracellular degradation of cAMP, a combination of cAMP or an analogous compound thereof and a substance that promotes intracellular synthesis of cAMP, a combination of a substance that suppresses intracellular degradation of cAMP and a substance that promotes intracellular synthesis of cAMP, or a combination of cAMP or an analogous compound thereof, a substance that suppresses intracellular degradation of cAMP, and a substance that promotes intracellular synthesis of cAMP.

Specifically, a combination of cAMP or an analogous compound thereof and CTAD may be used. In aspects according to the invention at least an agent which is cAMP or an analogous compound thereof selected from cAMP analogs, cGMP and cGMP analogs is present.

From the viewpoint of improving the capture rate of rare cells, the blood collection tube according to the present disclosure may further contain an anticoagulant agent or a composition containing the anticoagulant agent. The anticoagulant agent is as described above, and the amount of the anticoagulant agent to be contained in the blood collection tube may be such that the above-described final concentration of the anticoagulant agent is achieved when a predetermined amount of collected blood is introduced into the blood collection tube.

Therefore, in one or more examples, the present disclosure relates to a blood collection tube for use in a test for rare cells in blood, containing an agent that increases the intracellular concentration of cAMP or an analogous compound thereof, and an anticoagulant agent.

From the viewpoint of improving the capture rate of rare cells, the blood collection tube according to the present disclosure may further contain a COX inhibitor or a composition containing the COX inhibitor. The COX inhibitor is as described above, and the amount of the COX inhibitor to be contained in the blood collection tube may be such that the above-described final concentration of the COX inhibitor is achieved when a predetermined amount of collected blood is introduced into the blood collection tube.

Therefore, in one or more examples, the present disclosure relates to a blood collection tube for use in a test for rare cells in blood, containing an agent that increases the intracellular concentration of cAMP or an analogous compound thereof, and a COX inhibitor.

In one or more other examples, the present disclosure relates to a blood collection tube for use in a test for rare cells in blood, containing an agent that increases the intracellular concentration of cAMP or an analogous compound thereof, an anticoagulant agent, and a COX inhibitor.

From the viewpoint of improving the capture rate of rare cells, the blood collection tube according to the present disclosure may further contain glucose or a composition containing glucose. The glucose or the composition containing glucose is as described above, and the amount of glucose to be contained in the blood collection tube may be such that the above-described final concentration of glucose is achieved when a predetermined amount of collected blood is introduced into the blood collection tube.

Therefore, in one or more examples, the present disclosure relates to a blood collection tube for use in a test for rare cells in blood, containing an agent that increases the intracellular concentration of cAMP or an analogous compound thereof, and glucose.

In one or more other examples, the present disclosure relates to a blood collection tube for use in a test for rare cells in blood, containing an agent that increases the intracellular concentration of cAMP or an analogous compound thereof, an anticoagulant agent, and glucose.

In one or more still other examples, the present disclosure relates to a blood collection tube for use in a test for rare cells in blood, containing an agent that increases the intracellular concentration of cAMP or an analogous compound thereof, an anticoagulant agent, a COX inhibitor, and glucose.

From the viewpoint of improving the capture rate of rare cells, the blood collection tube according to the present disclosure may further contain a granulocyte elastase inhibitor or the composition containing the granulocyte elastase inhibitor. The granulocyte elastase inhibitor or the composition containing the granulocyte elastase inhibitor is as described above, and the amount of the granulocyte elastase inhibitor to be contained in the blood collection tube may be such that the above-described final concentration of the granulocyte elastase inhibitor is achieved when a predetermined amount of collected blood is introduced into the blood collection tube.

Therefore, in one or more examples, the present disclosure relates to a blood collection tube used in a test for rare cells in blood, containing an agent that increases the intracellular concentration of cAMP or an analogous compound thereof, and a granulocyte elastase inhibitor.

In one or more other examples, the present disclosure relates to a blood collection tube for use in a test for rare cells in blood, containing an agent that increases the intracellular concentration of cAMP or an analogous compound thereof, an anticoagulant agent, and a granulocyte elastase inhibitor.

In one or more still other examples, the present disclosure relates to a blood collection tube for use in a test for rare cells in blood, containing an agent that increases the intracellular concentration of cAMP or an analogous compound thereof, an anticoagulant agent, a COX inhibitor, and a granulocyte elastase inhibitor.

In one or more still other examples, the present disclosure relates to a blood collection tube for use in a test for rare cells in blood, containing an agent that increases the intracellular concentration of cAMP or an analogous compound thereof, an anticoagulant agent, a COX inhibitor, glucose, and a granulocyte elastase inhibitor.

From the viewpoint of improving the efficiency of a rare cell test, the blood collection tube according to the present disclosure may further contain an antioxidant agent or a composition containing the antioxidant agent. The antioxidant agent or the composition containing the antioxidant agent is as described above, and the amount of the antioxidant agent to be contained in the blood collection tube may be such that the above-described final concentration of the antioxidant agent is achieved when a predetermined amount of collected blood is introduced into the blood collection tube.

Therefore, in one or more examples, the present disclosure relates to a blood collection tube for use in a test for rare cells in blood, containing an agent that increases the intracellular concentration of cAMP or an analogous compound thereof, and an antioxidant agent.

In one or more other examples, the present disclosure relates to a blood collection tube for use in a test for rare cells in blood, containing an agent that increases the intracellular concentration of cAMP or an analogous compound thereof, an anticoagulant agent, and an antioxidant agent.

In one or more still other examples, the present disclosure relates to a blood collection tube for use in a test for rare cells in blood, containing an agent that increases the intracellular concentration of cAMP or an analogous compound thereof, an anticoagulant agent, a COX inhibitor, and an antioxidant agent.

In one or more still other examples, the present disclosure relates to a blood collection tube for use in a test for rare cells in blood, containing an agent that increases the intracellular concentration of cAMP or an analogous compound thereof, an anticoagulant agent, a COX inhibitor, glucose, and an antioxidant agent.

In one or more still other examples, the present disclosure relates to a blood collection tube for use in a test for rare cells in blood, containing an agent that increases the intracellular concentration of cAMP or an analogous compound thereof, an anticoagulant agent, a COX inhibitor, glucose, a granulocyte elastase inhibitor, and an antioxidant agent.

In one or more examples, the blood collection tube according to the present disclosure may contain one or more other components as long as the other components do not greatly interfere with the rare cell test. In other examples, the present disclosure relates to use of a blood collection tube in a test for rare cells in blood, wherein the blood collection tube (including a blood collection tube for use in a test for rare cells in blood) is as described above. The test for rare cells may be as described above.

Blood Collection Tube (Second Aspect)

The blood collection tube according to the present disclosure may be used in blood component tests other than the rare cell test. This is because the blood collection tube according to the present disclosure stabilizes blood components. Therefore, the present disclosure also relates to a blood collection tube (second aspect) containing an agent that increases the intracellular concentration of cAMP or an analogous compound thereof. In the blood collection tube of the present present disclosure, blood can be stabilized by cAMP or an analogous compound thereof.

As to the agent that increases the intracellular concentration of cAMP or an analogous compound thereof and other agents (anticoagulant agent, COX inhibitor, glucose, granulocyte elastase inhibitor, antioxidant agent, etc.) to be mixed with the blood, reference can be made to the descriptions regarding these agents in the above-described blood collection tube according to the present disclosure. In aspects according to the invention the agent that increases the intracellular concentration of cAMP is cAMP or an analogous compound thereof selected from cAMP analogs, cGMP and cGMP analogs.

In one or more examples, the blood collection tube according to the present disclosure (second aspect) may be a blood collection tube containing an agent that increases the intracellular concentration of cAMP or an analogous compound thereof and a COX inhibitor.

In one or more other examples, the blood collection tube according to the present disclosure (second aspect) may be a blood collection tube containing cAMP or an analogous compound thereof.

The blood collection tube according to the present disclosure (second aspect) may be in the form of a blood collection bag.

Examples

Hereinafter, the present disclosure will be described more specifically by way of examples and comparative examples. However, they are merely illustrative and the present disclosure should not be interpreted as being limited to the following examples.

Model samples of CTC-containing blood were prepared using whole blood and cancer cells collected from a human. Thereafter, the cancer cells in each of the model samples were captured by a filter. The CTCs on the filter were counted using a microscope, and the capture rate was calculated. In Experiments 1 to 4 to be described below, CTC separation was performed according to the method disclosed in JP 2016-180753A and using the following filter under the following filtration conditions. As CTC model cells, SW620 was used as a sample of small-sized cancer cells, and SNU-1 was used as a sample of cancer cells with high deformability. Even when stored blood is used, it is possible to achieve a high capture rate by controlling the amount of blood as appropriate. The appropriate volume of the blood for the high capture rate can be set by converting to the amount of blood per hole area of the filter.

Filter

The following filter was used as a filter for capturing CTCs (cancer cells).

Hole shape: slit shape

$$\text{Minor axis diameter } (\mu m) \times \text{Major axis diameter } (\mu m) = 6.5 \times 88$$

Hole area $(\mu m^2)$ = 572
Pitch between centers of holes:

$$(\text{minor axis diameter side} \times \text{major axis diameter side}) \, (\mu m) = 14 \times 100$$

Hole density (the number of holes/mm$^2$) = 714
Aperture ratio (%) = 41
Thickness $(\mu m)$ = 5
Material: nickel
Filtration area (mm$^2$) = 20
Total number of holes = 14025

Filtration Conditions

1. The blood sample was fed through the filter from a tube pump at a filtration speed of 100 $\mu l$/min.
2. First washing: EDTA-containing PBS (-) solution 2 ml 100 $\mu l$/min
3. Second washing: EDTA-containing PBS (-) solution 2 ml 1000 $\mu l$/min
4. Third washing: EDTA-containing PBS (-) solution 2 ml 1000 $\mu l$/min
5. After the washing, the filter surface was observed with a microscope.

Preparation of Small-Sized Cancer Cell Sample SW620 Sample

According to a commonly used procedure, human colon cancer cells (SW620 cell line), which are adherent cells, were cultured in a petri dish, the culture supernatant was then removed, the medium components were further removed by adding PBS (-) for washing, and thereafter, the cells were treated (at 37°C for 3 minutes) with trypsin (Invitrogen) (37°C, 3 minutes). A serum-containing medium was added thereto, and the resultant mixture was collected into a 1.5 ml Eppendorf tube. This was centrifuged at 300 $\times$ g and ordinary temperature (24°C) for 3 minutes using a small-sized centrifuge, and the supernatant was removed. The cells were again suspended in a serum-containing medium and collected. Thus, a cancer cell suspension was prepared.

Next, the cancer cells were subjected to fluorescent staining. The cancer cells were collected from the prepared cancer cell suspension, and were then centrifuged at 300 $\times$ g and ordinary temperature (24°C) for 3 minutes using a small-sized centrifuge. The supernatant was removed, and PBS (-) was added to suspend the cancer cells. The cancer cells suspended in PBS (-) were subjected to fluorescent staining using a staining reagent CellTracker (Invitrogen). The CellTracker was dissolved in DMSO such that the concentration thereof was 10 mM. Thereafter, the resultant mixture was added to the cell suspension such that the final concentration of the CellTracker during the reaction was 0.5 $\mu M$ to 0.25 $\mu M$. The reaction was allowed to proceed at 37°C for 30 minutes. Thereafter, the cell suspension was centrifuged, the supernatant was removed, and the residue was suspended in PBS again. These operations were repeated to remove the unreacted solution and to wash the cells. Thereafter, the cells were suspended in a culture medium or PBS (-) such that the cell suspension had a desired concentration (about $10^3$ cells/ml to about 5 $\times$ $10^4$ cells/ml).

Preparation of Sample of Cancer Cells with High Deformability SNU-1 Sample

Human stomach cancer cells (SNU-1 cell line), which are floating cells, were collected into a 1.5 ml Eppendorf tube without being treated with trypsin. The fluorescent staining was performed in the same manner as in the preparation of the SW620 sample, except that the collected cancer cell suspension was used. Although the SNU-1 cells have a cell diameter of 16.8 $\mu m$, the SNU-1 cells passed through a filter with holes having a diameter of 6.5 $\mu m$ more easily than the SW620 cells (cell diameter: 13 $\mu m$).

Blood Collection Tubes

The following commercially available blood collection tubes were used as blood collection tubes.

CTAD blood collection tube: a blood collection tube containing a mixture solution of citrate, theophylline, adenosine, and dipyridamole (Nippon Becton Dickinson Company, Ltd.)

Citrate blood collection tube: a blood collection tube containing a solution of sodium citrate in an amount to achieve a final concentration of 0.32% (Terumo Corporation)

EDTA2K blood collection tube: (Terumo Corporation)

Heparin Na blood collection tube: (Terumo Corporation)

ACD blood collection tube: a blood collection tube containing a mixture solution of a sodium citrate hydrate, a citric acid hydrate, and glucose (Nippon Becton Dickinson Company, Ltd.)


Reagents to be Added to Blood Collection Tube

DBcAMP: (dibutyl cAMP, Tokyo Chemical Industry Co., Ltd., 100 mM) (cAMP analog) 8-pCPT-cAMP: (8-(4-chlorophenylthio)-cAMP, Wako Pure Chemical Industries, Ltd., 50 mM) (cAMP analog)
CFC: (solvent: mixture of ethanol and water [ethanol : water = 2 :1 ])

caffeine (Wako Pure Chemical Industries, Ltd., 101 mM) (xanthine, PDE inhibitor)
forskolin (Tokyo Chemical Industry Co., Ltd., 24.8 mM) (cAMP synthesis promoter)
cilostazol (Wako Pure Chemical Industries, Ltd., 13.2 mM) (PDE3 specific inhibitor)

R: (rolipram, Tokyo Chemical Industry Co., Ltd., 20 mM in EtOH) (PDE4 specific inhibitor)
D: (dipyridamole, Wako Pure Chemical Industries, Ltd., 19.8 mM in EtOH) (PDE5, PDE7, and PDE8 specific inhibitor)
ASP (1): (aspirin, Wako Pure Chemical Industries, Ltd., 1.1 M in EtOH) (COX inhibitor)
ASP (2): (aspirin, Wako Pure Chemical Industries, Ltd., 111 mM in EtOH) (COX inhibitor)
CPDA:

sodium citrate hydrate (Nacalai Tesque, Inc.,109 mM)
citric acid monohydrate (Nacalai Tesque, Inc., 20 mM)
sodium dihydrogenphosphate (Nacalai Tesque, Inc., 2 mM)
D-(+)-glucose (Nacalai Tesque, Inc., 188 mM)
adenine (Nacalai Tesque, Inc., 2 mM)

MADI:

D-mannitol (Nacalai Tesque, Inc., 197 mM)
adenine (Wako Pure Chemical Industries, Ltd., 2.95 mM)
D-(+)-glucose (Nacalai Tesque, Inc., 100 mM)
inosine-5-monophosphate disodium salt (Wako Pure Chemical Industries, Ltd., 40 mM)

Sivelestat: (Sigma-Aldrich Co., about 23 mM) (granulocyte elastase inhibitor)
SS-31: (product name: HY-P125, MedChemExpress, 10 mM in DMSO) (antioxidant agent)
NaCl (1): (1.8% in water)
NaCl (2): (0.9% in water)


Experiment 1

In order to eliminate the need to consider the aging of the cancer cells over time, model samples were prepared by adding, to whole blood stored at ordinary temperature for 24 hours or 48 hours, cancer cell samples that had been prepared on the day when the model samples were fed through the filter to separate the cancer cells. With this experiment, the influence of the change in blood components due to fresh blood being stored at ordinary temperature for 24 hours or 48 hours was evaluated.

Specifically, the experiment was performed in the following manner. Blood was collected from a human to blood collection tubes (commercially available blood collection tubes). Then, 9 to 10 ml of whole blood was aliquoted into 15 ml centrifuge tubes, which were used for preparation of model samples. Reagents shown in Table 1 below were added to the aliquots of the blood. The resultant mixtures were stored at ordinary temperature for 24 hours or 48 hours, and thereafter, about 300 cancer cells (SNU-1 and SW620) prepared immediately before subjecting the blood to filtration were added thereto. Model samples were prepared (Examples 1 to 3 and Comparative Examples 1 to 5) in this manner.

Out of each of the thus-prepared model samples, an amount equivalent to 8 ml was filtered through the filter under the above-described filtration conditions to capture the cancer cells, and the capture rate was calculated. Specifically, out of the model sample (10 ml) prepared by adding the reagent shown in Table 1 such that the total amount of the blood and the reagent would be about 10 ml, the amount of the model sample to be subjected to the filtration was adjusted such that the filtration amount would be 8 ml (for example, in the case of the model sample obtained by adding 1 ml of the reagent in total to 9 ml of the whole blood aliquoted from the blood collection tube to the centrifuge tube, 8.9 ml of the model sample was used. In the case of the model sample obtained by adding 200 μl of the reagent in total to 10 ml of the whole blood aliquoted from the blood collection tube to the centrifuge tube, 8.2 ml of the model sample was used). The results obtained are shown in Table 1 below

| Table 1 | Blood collection tube | Amount added to blood collection tube (μl) (per 9 to 10 ml of collected blood) | | | | | | | SNU-1 capture rate | | SW620 capture rate | |
|---|---|---|---|---|---|---|---|---|---|---|---|---|
| | | DBcAMP | ASP (1) | CPDA | MADI | Sivelestat | SS-31 | NaCl (1) | 24 h | 48 h | 24 h | 48 h |
| Comp. Ex. 1 | citrate | - | - | - | - | - | - | - | 26% | 10% | 74% | 73% |
| Ex. 1 | citrate | 100 | - | - | - | - | - | 100 | 50% | - | 91% | - |
| Ex. 2 | CTAD | - | - | - | - | - | - | - | 72% | 44% | 92% | 76% |
| Ex. 3 | CTAD | - | - | - | 1000 | - | - | - | - | 54% | - | 88% |
| Comp. Ex. 2 | EDTA-2K | - | - | - | - | - | - | - | 14% | - | 34% | - |
| Comp. Ex. 3 | EDTA-2K | - | 10 | 1000 | - | 10 | 1 | - | 19% | - | 39% | - |
| Comp. Ex. 4 | Heparin Na | - | - | - | - | - | - | - | 24% | - | 74% | - |
| Comp. Ex. 5 | ACD | - | - | - | - | - | - | - | 6% | - | 7% | - |

When collected blood is stored for a long time at ordinary temperature, for example, the blood is likely to clog a filter owing to the formation of aggregates as a result of the deterioration in deformability of erythrocytes and the cell death of leukocytes, formation of aggregates by platelets etc., and the like. In the case where fresh blood is used, after a blood sample obtained by adding SNU-1 cells or SW620 cells to the fresh blood is passed through a filter, at least 90% of the SNU-1 cells and SW620 cells remain on the filter (i.e., captured by the filter). In contrast, in the case where a blood sample that has been stored for a long time at ordinary temperature is used, the pressure difference above and below the filter tends to be large. As a result, cancer cells pass through the filter more easily, and the capture rate of the cancer cells is reduced even if the blood sample contains the cancer cells prepared immediately before filtration is performed. The results obtained regarding Comparative Examples 1 to 5 in which the conventional anticoagulant agents were used also suggest the occurrence of this phenomenon.

In contrast, in Example 1 in which DBcAMP was added and in Examples 2 and 3 in which the CTAD blood collection tubes were used, the capture rates were improved as compared with those in Comparative Examples 1 to 5. DBcAMP supplies cAMP when it is degraded in cells. Also, it has been reported that DBcAMP itself inhibits phosphodiesterase. That is, the above results demonstrate that, in an environment where the intracellular concentration of cAMP in blood cells is increased, the reduction in the capture rate caused by the change in blood components, e.g., the change in blood cells (in other words, not caused by the change in CTCs to be separated) due to the fresh blood being stored at ordinary temperature can be suppressed. Similarly, it is considered that, in the case where CTAD was added, not only the action of CTAD on platelets but also an increase in the intracellular concentration of cAMP in leukocytes and the like served to improve the capture rate. It was thus found that a CTAD blood collection tube can be used suitably for rare cell tests. In particular, in the case where a rare cell test is performed after storing blood for a long time (e.g., at least 4 hours or at least 24 hours), the effect of using the CTAD blood collection tube is more prominent. Whether the change in blood components is suppressed can be checked by, particularly in the case where leukocytes are to be measured, checking whether the histogram of the bare-nucleated leukocytes is stable using a Coulter blood cell counter such as SB1440, for example.

In Example 3 in which MADI containing glucose that synthesizes and supplies ATP was added, the capture rate of the cancer cells in the blood sample stored for 48 hours was improved as compared with that in Example 2 in which MADI was not added.

SNU-1 and SW620 are cancer cells that cannot be captured easily by a filter. Accordingly, if these cancer cells can be captured, it is expected that various types of cancer cells can be captured.

Experiment 2

Model samples were prepared by adding cancer cells to whole blood and then storing the mixtures at ordinary temperature for 24 or 48 hours. Then, the cancer cells were separated using the filter, and the capture rates were evaluated.

Unlike Experiment 1, Experiment 2 evaluated the influence of not only the change in blood components resulting from storage at ordinary temperature for 24 hours or 48 hours but also the change in the cancer cells resulting from storage at ordinary temperature for 24 hours or 48 hours on the capture rate.

Blood (10 ml in total with the solution in the blood collection tube) was collected from a human into a blood collection tube (citrate blood collection tube or CTAD blood collection tube), and aliquoted into 15 ml centrifuge tubes. Reagents shown in Table 2 below were added to the aliquots of the blood, and about 300 of the prepared cancer cells (SNU-1 and SW620) were added thereto. Thereafter, the resultant mixtures were stored at ordinary temperature for 24 hours or 48 hours. Model samples were prepared (Examples 4 to 6 and Comparative Examples 6 to 7) in this manner. With this experiment, the influence of storage of fresh blood containing the cancer cells was evaluated.

Out of each of the thus-prepared model samples, an amount equivalent to 8 ml was filtered through the filter under the above-described filtration conditions to capture the cancer cells, and the capture rate was calculated. The results obtained are shown in Table 2 below.

| Table 2 | Blood collection tube | Amount added to blood collection tube (µl) (per 9 to 10 ml of collected blood) | | | SNU-1 capture rate | | SW620 capture rate | |
|---|---|---|---|---|---|---|---|---|
| | | DBcAMP | CPDA | NaCl (1) | 24 h | 48 h | 24 h | 48 h |
| Comp. Ex. 6 | citrate | - | - | - | 23% | - | 75% | - |
| Comp. Ex. 7 | citrate | - | 1000 | - | 12% | 2% | 67% | 19% |

(continued)

| Table 2 | Blood collection tube | Amount added to blood collection tube (μl) (per 9 to 10 ml of collected blood) | | | SNU-1 capture rate | | SW620 capture rate | |
|---|---|---|---|---|---|---|---|---|
| | | DBcAMP | CPDA | NaCl (1) | 24 h | 48 h | 24 h | 48 h |
| Ex. 4 | citrate | 100 | - | 100 | 26% | - | 100% | - |
| Ex. 5 | citrate | 100 | 1000 | - | 57% | 8% | 89% | 63% |
| Ex. 6 | CTAD | - | - | - | 55% | 10% | 93% | 16% |

In Examples 4 and 5 in which DBcAMP was added and in Example 6 in which the CTAD blood collection tube was used, the capture rates were improved as compared with that in Comparative Example 6 in which none of the reagents were added. That is, an improvement in the capture rate was observed under the conditions where the intracellular concentration of cAMP was increased. Regarding the model samples stored for 24 hours, as a result of comparing Examples 4 and 5 with Comparative Example 7, it was found that, by adding DBcAMP, the capture rate of SNU-1 was improved, and further, by adding both DBcAMP and CPDA, the capture rate of SNU-1 was improved to about double the capture rate when DBcAMP alone was added. In the model samples stored for 48 hours, the capture rate of the cancer cells in the stored blood sample obtained by storing the blood after adding the cancer cells thereto (Table 2, Example 6) was reduced as compared with that in the stored blood sample obtained by adding cancer cells that were prepared immediately before filtration and had little deterioration to the stored blood (Table 1, Example 2). Although the details of the mechanism by which the capture rate was reduced have not been clarified, it is considered that, when the blood was stored in the state where the cancer cells were contained therein, the capture rate was reduced owing to a change in the cancer cells over time. As in Example 5, when CPDA was added under the conditions where cAMP and the cAMP analogs in cells were increased, the capture rate of the cancer cells in the model sample stored for 48 hours was improved as compared with that in Comparative Example 7.
The capture rate in Comparative Example 7 in which only CPDA was added did not differ very much from the capture rate in Comparative Example 6.

Experiment 3 (Comparative Experiment)

In order to eliminate the need to consider the aging of cancer cells over time, model samples were prepared by adding, to whole blood stored at 4°C for 72 hours, cancer cell samples that had been prepared on the day when the model samples were subjected to separation by filtration. With this experiment, the influence of the storage of fresh blood at 4°C for 72 hours was evaluated.
Blood (10 ml in total with the solution in the blood collection tube) was collected from a human into a CTAD blood collection tube, and the collected blood was aliquoted into 15 ml centrifuge tubes. Reagents shown in Table 3 below were added the aliquots of the blood, and the resultant mixtures were stored at 4°C for 72 hours. Thereafter, the aliquots of the blood were brought to ordinary temperature, and about 300 of the prepared cancer cells (SNU-1 and SW620) were added thereto. Model samples were prepared (Examples 7 to 8) in this manner.
8 ml of each of the thus-prepared model samples was filtered through the filter under the above-described filtration conditions to capture the cancer cells, and the capture rate was calculated. The results obtained are shown in Table 3 below.

| Table 3 | Blood collection tube | Amount added to blood collection tube (μl) (per 9 to 10 ml of collected blood) | | SNU-1 capture rate | SW620 capture rate | |
|---|---|---|---|---|---|---|
| | | ASP (2) | NaCl (1) | 72 h | 72 h | |
| Ex. 7 | CTAD | - | - | 6% | 23% | *4°C 72 h |
| Ex. 8 | CTAD | 10 | 10 | 33% | 86% | *4°C 72 h |

As can be seen in Table 3, in Example 8 in which aspirin (ASP) was added, the capture rates of SNU-1 and SW620 were both improved as compared with those in Example 7 in which ASP was not added. Also, it was found through visual observation that, while the formation of large aggregates was observed on the filter in Example 7, the formation of large aggregates was suppressed in Example 8.

As compared with the case where the CTAD blood collection tube (Table 1, Example 2) was stored at room temperature for 24 hours, when the cancer cells were captured by the filter after the CTAD blood collection tube had been stored in a refrigerated state for 24 hours and then brought to room temperature, the capture rate of SNU-1 was reduced from 72% to 32% and the capture rate of SW620 was reduced from 92% to 54%.

Although the details of the mechanism have not been clarified, it is considered that, while storage at low temperatures adversely affects blood, the influence of the change in blood components during the storage at low temperatures was suppressed by further adding ASP in addition to CTAD, whereby a high capture rate could be achieved even if the blood was treated after being brought to room temperature.

Experiment 4

Unlike Experiment 3, Experiment 4 evaluated the influence of not only the change in blood components resulting from storage at 4°C for 72 hours but also the change in the cancer cells resulting from storage at 4°C for 72 hours on the capture rate.

Blood (10 ml in total with the solution in the blood collection tube) was collected from a human into a blood collection tube (citrate blood collection tube or CTAD blood collection tube), and aliquoted into 15 ml centrifuge tubes. Reagents shown in Table 4 below were added to 9 ml of the aliquots of the blood, and about 300 cancer cells (SNU-1 and SW620) that had been prepared immediately before being added were added thereto. Thereafter, the resultant mixtures were stored at 4°C for 72 hours. Model samples were prepared (Examples 9 to 18 and Comparative Example 8) in this manner.

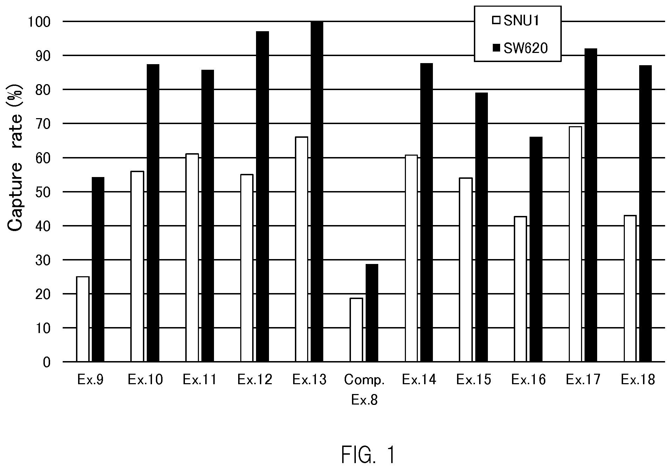

The model samples were brought to ordinary temperature. Then, 9 ml of each of the thus-prepared model samples was filtered through the filter under the above-described filtration conditions to capture the cancer cells, and the capture rate was calculated. The results obtained are shown in Table 4 below and FIG. 1.

| Table 4 | Blood collection tube | Amount added to blood collection tube (μl) (per 9 to 10 ml of collected blood) | | | | | | | | | | SNU-1 capture rate | SW620 capture rate |
|---|---|---|---|---|---|---|---|---|---|---|---|---|---|
| | | DBcAMP | 8-pCPT-cAMP | CFC | D | R | ASP (2) | CPDA | Sivelestat | SS-31 | NaCl (2) | 72 h | 72 h |
| Ex. 9 | CTAD | - | - | - | - | - | 10 | - | - | - | 1000 | 25% | 54% |
| Ex. 10 | CTAD | - | - | - | - | - | 10 | 1000 | - | - | - | 56% | 87% |
| Ex. 11 | CTAD | - | - | - | - | - | 10 | 1000 | 10 | - | - | 61% | 86% |
| Ex. 12 | CTAD | - | - | - | - | - | 10 | 1000 | 10 | 1 | - | 55% | 97% |
| Ex. 13 | CTAD | 100 | - | - | - | - | 10 | 1000 | 10 | 1 | - | 66% | 104% |
| Comp. Ex. 8 | citrate | - | - | - | - | - | 10 | 1000 | 10 | - | - | 19% | 29% |
| Ex. 14 | citrate | 100 | - | - | - | - | 10 | 1000 | 10 | - | - | 61% | 88% |
| Ex. 15 | citrate | - | 200 | - | - | - | 10 | 1000 | 10 | - | - | 54% | 79% |
| Ex. 16 | citrate | - | - | 75 | - | - | 10 | 1000 | 10 | - | - | 43% | 66% |
| Ex. 17 | citrate | - | - | 75 | 10 | - | 10 | 1000 | 10 | - | - | 69% | 92% |
| Ex. 18 | citrate | - | - | 75 | - | 10 | 10 | 1000 | 10 | - | - | 43% | 87% |

As can be seen in Table 4 and FIG. 1, improvement in the capture rate was observed by adding CPDA, which is used as a blood stabilizer, an ATP supplying agent, and/or a nutrient (Example 9 vs Example 10). Also, improvement in the capture rate was observed by adding sivelestat, which is a granulocyte (neutrophil) elastase inhibitor (Example 10 vs Example 11). Furthermore, improvement in the capture rate of the SW620 cells, which proliferate by adhesion, was observed by adding SS-31, which is an antioxidant agent (Example 11 vs Example 12). Comparison between Example 12 and Example 13 revealed that the capture rates in Example 13 were improved as compared to those in Example 12, and besides, in Example 13 in which DBcAMP was added, the filter surface after filtration was clean with only a few aggregates remaining thereon, which facilitated analysis.

By comparing Examples 14 to 18 with Comparative Example 8, it can be seen that, in a test for rare cells in blood, it is important to add an agent that increases the intracellular concentration of cAMP. In the samples collected from the filter, many small aggregates of the blood components were observed in Comparative Example 8, whereas the formation of small aggregates was suppressed in Example 14. From the results obtained when the phosphodiesterase inhibitors were added, it was found that the capture rates were improved remarkably by adding the substances that inhibit the phosphodiesterases specific to immunocytes in addition to the inhibitors specific to the phosphodiesterases in platelets (Examples 16, 17, and 18).

Experiment 5

Analysis of RNAin Rare Cells

Blood was collected from a human into a blood collection tube (CTAD blood collection tube), and 9 ml of whole blood in the blood collection tube was aliquoted into 15 ml centrifuge tubes. These aliquots were used for preparation of model samples. The same reagents as in Examples 11 and 12 (Table 4) were mixed with 9 ml of the aliquots of the blood. Cancer cells (SKBR3) that had been prepared and stained in the same manner as the above-described SNU-1 sample and SW620 sample immediately before being added were added thereto. Thereafter, the resultant mixtures were stored at 4°C for 72 hours. Model samples were prepared in this manner. 9 ml of each of the model samples was filtered through a filter having the same configuration as the above-described filter except that the filtration area (the area of the filter) was 28 mm$^2$, and then was subjected to washing. The filtration speed of the filtration and the washing were both performed at 1000 $\mu$l/min. After the filtration and the washing, a commercially available hemolytic agent was fed through the filter. Erythrocytes remaining on the filter were hemolyzed by allowing the reaction to proceed for 10 minutes, and then, the filter was washed again by feeding a PBS (-) solution through the filter. The SKBR3 cells were collected from the filter by causing the PBS (-) solution to reversely feed (backflow), and the number of collected cells was estimated by using a microscope to count the number of cells in a portion of the collected sample. The recovery rate of the SKBR3 cells collected by causing the back flow through the filter was 71% when either the reagent in Example 11 or the reagents in Example 12 were added.

The remaining part of the collected sample was purified using an RNA purification kit RNeasy Mini Kit (Cat No./ID: 74104, QIAGEN K. K.). Thereafter, using an ABI7500, RT-PCR analysis of mRNA of ERBB2 of the SKBR3 cells was performed using a One-Step qRT-PCR Kit w/ROX (Product No.: 11745100, Thermo Fisher Scientific K.K.) and a commercially available probe for ERBB2 (Assay ID: Hs01001580_m1, Thermo Fisher Scientific K.K.).

In the analysis, two types of references were provided. The two types of references were as follows: the reference obtained by scraping the cultured SKBR3 cells from the petri dish according to a commonly used procedure and then subjecting the SKBR3 cells to an adjustment treatment such that the number of the SKBR3 cells reached a desired value within 1 hour (Reference A); and a RPMI medium alone (Reference B). Using these references, RNA extraction and RT-PCR analysis on mRNA of ERBB2 of the SKBR3 cells were performed in the same manner as in the above.

In RT-PCR, DNAis synthesized with RNA as a template, and the thus-synthesized DNA is amplified exponentially in such a manner that the number of synthesized DNA sequences is doubled in each PCR cycle. The $C_T$ value indicates the number of cycles required for the PCR amplification product to reach a certain amount. The smaller the number of cycles, i.e., the smaller the $C_T$ value, the more mRNA is present in the sample at the start of RTPCR. When there is no difference in the number of cells, the closer the $C_T$ value to that of Reference A immediately after being cultured, the more mRNA remains in the SKBR3 cells in the collected sample.

As a result of the RT-PCR analysis, in Reference B (RPMI medium alone), the $C_T$ value was not obtained and mRNA of ERBB2 was not detected. In Reference A, the number of SKBR3 cells was about 460, and the CT value was 25.22.

In about 460 SKBR3 cells out of the SKBR3 cells collected from the blood samples prepared in the same manner as in Examples 11 and 12, the $C_T$ value was 25.98 in Example 11 and 26.56 in Example 12. In the sample collected after filtering only the blood through the filter (i.e., the sample without SKBR3), the $C_T$ value was 35.77, and this suggested that a small amount of mRNA of ERBB2 was present in the blood.

The $C_T$ value of the collected sample obtained from the blood containing SKBR3 was obviously smaller than that of the collected sample obtained from the blood without SKBR3, and mRNA of ERBB2 of SKBR3 could be detected. The results demonstrate that, even after storage at 4°C for 72 hours, mRNA of ERBB2 in SKBR3 still remained and was detectable.

FISH Analysis of Rare Cells

Blood was collected from a human into a blood collection tube (citrate blood collection tube), and 9 ml of whole blood in the blood collection tube was aliquoted into 15 ml centrifuge tubes. These aliquots were used for preparation of model samples. The same reagents as in Example 14 were mixed with 9 ml of the aliquots of the blood. Several thousand cancer cells (SKBR3) prepared in the same manner as the above-described SNU-1 sample and SW620 sample except that they were not stained beforehand were added thereto. Thereafter, the resultant mixtures were stored at 4°C for 72 hours. Model samples were prepared in this manner. 9 ml of each of the model samples was subjected to filtration under the same filter conditions as described above, and was then subjected to washing. The filtration speed of the filtration and the washing were both performed at 1000 $\mu$l/min. After the filtration and the washing, a commercially available hemolytic agent was fed through the filter. Hemolysis was caused by allowing the reaction to proceed for 10 minutes, and then, the filter was washed again by feeding a PBS (-) solution through the filter. The SKBR3 cells (breast cancer cells) on the filter were collected by causing the PBS (-) solution to reversely feed (backflow), and the collected SKBR3 cells were applied onto a microscope slide. For DNA-FISH, the cells were immobilized on a microscope slide with a Carnoy's solution. For RNA-FISH, after immobilizing the cells on a microscope slide, the cells were immobilized with PBS (-) containing 4% formaldehyde.

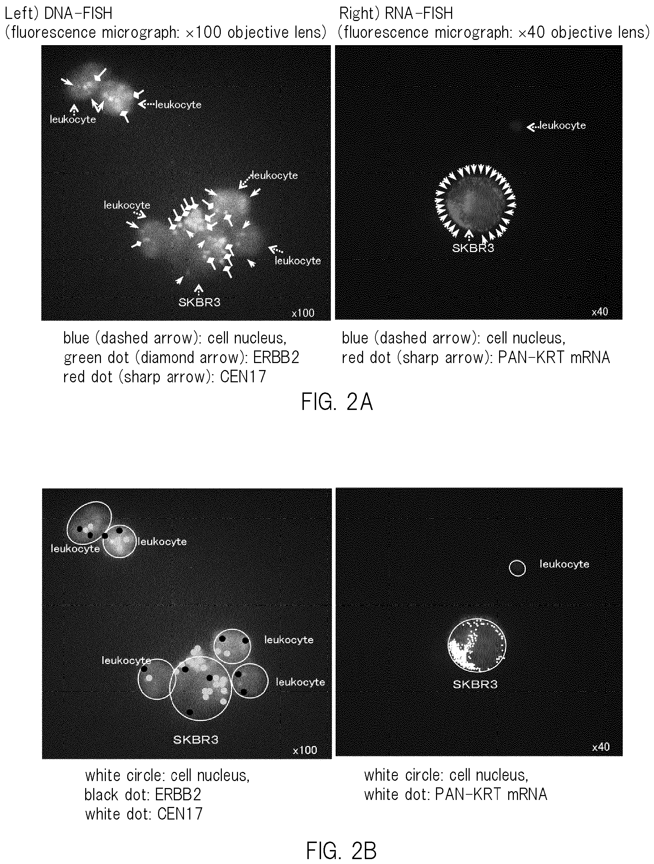

The DNA-FISH was performed in the following manner. In the same manner as in the above, the model sample was subjected to filtration and then a sample (SKBR3) was collected. According to a commonly used procedure of DNA-FISH, the collected sample was stained using a Zytolight® SPEC ERBB2/CEN17 Dual Color probe (ZytoVision GMbH), and the stained sample was applied and immobilized onto a microscope slide. As a result, the amplification of the ERBB2 gene was observed with a fluorescence microscope (on the left in FIGs. 2A and 2B).

The RNA-FISH was performed in the following manner. The collected sample (SKBR3) was treated using a ViewRNA Cell Assay Kit (TFA-QVC0001, Thermo Fisher Scientific K.K.). Then, in accordance with the protocol and the reagent composition thereof, the collected sample was further treated using a PAN-KRT (VA1-19147, Affymetrix, Inc.). The treated sample was applied and immobilized onto a microscope slide. As a result, the staining of mRNA of cytokeratin was observed (on the right in FIG. 2A and 2B).

According to the above-described procedures, the gene analysis of the samples after being stored at 4°C for 72 hours could be performed.

The embodiments disclosed in this application are to be considered in all respects as illustrative and not limiting. The scope of the invention is indicated by the appended claims rather than by the foregoing description.

## Claims

1. A method for treating collected blood to be used in a test for rare cells in blood, the method comprising:
   mixing the collected blood with cAMP or an analogous compound thereof selected from the group consisting of cAMP analogs, cGMP and cGMP analogs.

2. The method according to claim 1, wherein the cAMP analog is selected from 8-bromo-cAMP sodium salt, 6-monobutyryl cAMP, dibutyryl cAMP sodium salt, 8-(4-chlorophenylthio)-cAMP sodium salt, 8-(4-chlorophenylthio)-2'-O-methyl cAMP sodium salt, 2-chloro-8 methylamino cAMP, N-[6-(N-methylanthraniloylamino)hexyl]-cAMP and 8-(6-aminohexyl)amino-cAMP; and the cGMP analog is selected from 8-bromo-cGMP, dibutyryl-cGMP and 8-(4-chlorophenylthio)-guanosine 3',5'-cyclic monophosphate sodium salt.

3. The method according to claim 1 or 2, further comprising mixing the collected blood with an anticoagulant agent, preferably citrate.

4. The method according to any one of claims 1 to 3, further comprising mixing the collected blood with an agent that promotes introduction of the cAMP or the analogous compound thereof into a cell, wherein the agent is a surfactant, solvent or reagent that promotes endocytosis.

5. The method according to any one of claims 1 to 4, further comprising mixing the collected blood with a cyclooxygenase (COX) inhibitor.

**6.** The method according to any one of claims 1 to 5, further comprising mixing the collected blood with one selected from the group consisting of glucose, granulocyte elastase inhibitors, antioxidant agents, combinations thereof, and compositions comprising the same.

**7.** The method according to any one of claims 1 to 6, wherein the collected blood after the mixing is

(i) stored at a temperature of 10°C or less; and/or
(ii) stored for at least 4 hours.

**8.** A method for performing a test for rare cells in blood, the method comprising:

i) using blood treated by the method according to any one of claims 1 to 7 as a blood sample to be subjected to the test; or
ii) treating collected blood by a method according to any one of claims 1 to 7 and subjecting the treated collected blood to a test for rare cells.

**9.** The method of claim 8 wherein the test for rare cells comprises one or more of rare cell detection, rare cell separation, rare cell enrichment, rare cell measurement, rare cell observation, rare cell recovery, rare cell culture, and tests for DNA, RNA, a protein, or the like contained in rare cells.

**10.** A blood collection tube for use in a test for rare cells in blood, the blood collection tube comprising:
cAMP or an analogous compound thereof selected from the group consisting of cAMP analogs, cGMP and cGMP analogs.

**11.** The blood collection tube according to claim 10, wherein the cAMP analog is selected from 8-bromo-cAMP sodium salt, 6-monobutyryl cAMP, dibutyryl cAMP sodium salt, 8-(4-chlorophenylthio)-cAMP sodium salt, 8-(4-chlorophenylthio)-2'-O-methyl cAMP sodium salt, 2-chloro-8 methylamino cAMP, N-[6-(N-methylanthraniloylamino)hexyl]-cAMP and 8-(6-aminohexyl)amino-cAMP; and the cGMP analog is selected from 8-bromo-cGMP, dibutyryl-cGMP and 8-(4-chlorophenylthio)-guanosine 3',5'-cyclic monophosphate sodium salt.

**12.** The blood collection tube according to claim 10 or 11, further comprising one selected from the group consisting of anticoagulant agents, cyclooxygenase (COX) inhibitors, glucose, granulocyte elastase inhibitors, antioxidant agents, combinations thereof, and compositions comprising the same.

**13.** A method for treating collected blood, the method comprising:

(i) mixing the collected blood with cAMP or an analogous compound thereof and with a cyclooxygenase (COX) inhibitor; or
(ii) mixing the collected blood with cAMP and an analogous compound thereof, wherein the analogous compound of cAMP is as defined in claim 1 or 2.

**14.** A blood collection tube comprising:

(i) cAMP or an analogous compound thereof and
a cyclooxygenase (COX) inhibitor; or
(ii) cAMP or an analogous compound thereof,

wherein the analogous compound of cAMP is as defined in claim 1 or 2.

**15.** Use of a blood collection tube in a test for rare cells in blood, wherein the blood collection tube is as defined in any one of claims 10 to 12 or 14.

**Patentansprüche**

**1.** Verfahren zur Behandlung von gesammeltem Blut zur Verwendung in einem Test auf seltene Zellen im Blut, wobei das Verfahren umfasst:
Mischen des gesammelten Blutes mit cAMP oder einer analogen Verbindung davon, ausgewählt aus der Gruppe bestehend aus cAMP-Analoga, cGMP und cGMP-Analoga.

2. Verfahren nach Anspruch 1, wobei das cAMP-Analogon ausgewählt ist aus 8-Brom-cAMP-Natriumsalz, 6-Monobutyryl-cAMP, Dibutyryl-cAMP-Natriumsalz, 8-(4-Chlorphenylthio)-cAMP-Natriumsalz, 8-(4-Chlorphenylthio)-2'-O-methyl-cAMP-Natriumsalz, 2-Chlor-8-methylamino-cAMP, N-[6-(N-methylanthraniloylamino)hexyl]-cAMP und 8-(6-Aminohexy])amino-cAMP; und das cGMP-Analogon ausgewählt ist aus 8-Brom-cGMP, Dibutyryl-cGMP und 8-(4-Chlorphenylthio)-guanosin-3',5'-zyklischem Monophosphat-Natriumsalz.

3. Verfahren nach Anspruch 1 oder 2, das weiter Mischen des gesammelten Blutes mit einem gerinnungshemmenden Mittel, vorzugsweise Zitrat, umfasst.

4. Verfahren nach einem der Ansprüche 1 bis 3, das weiter Mischen des gesammelten Blutes mit einem Mittel umfasst, das die Einführung des cAMP oder der analogen Verbindung davon in eine Zelle fördert, wobei das Mittel ein Tensid, Lösungsmittel oder Reagenz ist, das Endozytose fördert.

5. Verfahren nach einem der Ansprüche 1 bis 4, das weiter Mischen des gesammelten Blutes mit einem Cyclooxygenase- (COX) Inhibitor umfasst.

6. Verfahren nach einem der Ansprüche 1 bis 5, das weiter Mischen des gesammelten Blutes mit einem ausgewählten aus der Gruppe, die aus Glucose, Granulozyten-Elastase-Inhibitoren, Antioxidationsmitteln, Kombinationen davon und Zusammensetzungen, die dasselbe umfassen, besteht.

7. Verfahren nach einem der Ansprüche 1 bis 6, wobei das gesammelte Blut nach dem Mischen

    (i) bei einer Temperatur von 10 °C oder weniger gelagert wird; und/oder
    (ii) mindestens 4 Stunden lang gelagert wird.

8. Verfahren zur Durchführung eines Tests auf seltene Zellen im Blut, wobei das Verfahren umfasst:

    i) Verwenden von Blut, das durch das Verfahren nach einem der Ansprüche 1 bis 7 behandelt wurde, als Blutprobe, die dem Test zu unterziehen ist; oder
    ii) Behandeln von gesammeltem Blut durch ein Verfahren nach einem der Ansprüche 1 bis 7 und Unterziehen des behandelten gesammelten Bluts einem Test auf seltene Zellen.

9. Verfahren nach Anspruch 8, wobei der Test auf seltene Zellen eines oder mehr umfasst von:
Erkennung seltener Zellen, Abscheidung seltener Zellen, Anreicherung seltener Zellen, Messung seltener Zellen, Beobachtung seltener Zellen, Wiederherstellung seltener Zellen, Kultivierung seltener Zellen und Tests auf DNA, RNA, ein Protein oder dergleichen, die in seltenen Zellen enthalten sind.

10. Blutsammelröhrchen zur Verwendung bei einem Test auf seltene Zellen im Blut, wobei das Blutsammelröhrchen umfasst:
cAMP oder eine analoge Verbindung davon, ausgewählt aus der Gruppe bestehend aus cAMP-Analoga, cGMP und cGMP-Analoga.

11. Blutsammelröhrchen nach Anspruch 10, wobei das cAMP-Analogon ausgewählt ist aus 8-Brom-cAMP-Natriumsalz, 6-Monobutyryl-cAMP, Dibutyryl-cAMP-Natriumsalz, 8-(4-Chlorphenylthio)-cAMP-Natriumsalz, 8-(4-Chlorphenylthio)-2'-O-methyl-cAMP-Natriumsalz, 2-Chlor-8-methylamino-cAMP, N-[6-(N-methylanthraniloylamino)hexyl]-cAMP und 8-(6-Aminohexy])amino-cAMP; und das cGMP-Analogon ausgewählt ist aus 8-Brom-cGMP, Dibutyryl-cGMP und 8-(4-Chlorphenylthio)-guanosin-3',5'-zyklischem Monophosphat-Natriumsalz.

12. Blutsammelröhrchen nach Anspruch 10 oder 11, weiter umfassend ein Mittel, ausgewählt aus der Gruppe bestehend aus Gerinnungshemmern, Cyclooxygenase- (COX) Inhibitoren, Glucose, Granulozyten-Elastase-Inhibitoren, Antioxidationsmitteln, Kombinationen davon und Zusammensetzungen, die dasselbe umfassen.

13. Verfahren zur Behandlung von gesammeltem Blut, wobei das Verfahren umfasst:

    (i) Mischen des gesammelten Blutes mit cAMP oder einer analogen Verbindung davon und mit einem Cyclooxygenase- (COX) Inhibitor; oder

(ii) Mischen des gesammelten Blutes mit cAMP und einer analogen Verbindung davon, wobei die analoge Verbindung von cAMP wie in Anspruch 1 oder 2 definiert ist.

**14.** Blutsammelröhrchen, umfassend:

(i) cAMP oder eine analoge Verbindung davon, und
einen Cyclooxygenase- (COX) Inhibitor; oder
(ii) cAMP oder eine analoge Verbindung davon,

wobei die analoge Verbindung von cAMP wie in Anspruch 1 oder 2 definiert ist.

**15.** Verwendung eines Blutsammelröhrchens in einem Test auf seltene Zellen im Blut, wobei das Blutsammelröhrchen wie in einem der Ansprüche 10 bis 12 oder 14 definiert ist.

**Revendications**

**1.** Procédé de traitement de sang prélevé destiné à être utilisé dans un test de cellules rares dans le sang, le procédé comprenant :
le mélange du sang prélevé avec de l'AMPc ou un composé analogue de celui-ci choisi dans le groupe constitué par des analogues de l'AMPc, du GMPc et des analogues du GMPc.

**2.** Procédé selon la revendication 1, dans lequel l'analogue de l'AMPc est choisi parmi le sel de sodium de 8-bromo-AMPc, le sel de sodium de 6-monobutyryl-AMPc, le sel de sodium du dibutyryl-AMPc, le sel de sodium de 8-(4-chlorophénylthio)-AMPc, le sel de sodium de 8-(4-chlorophénylthio )-2'-O-méthyl-AMPc, le 2-chloro-8 méthylamino-AMPc, le N-[6-(N-méthylanthraniloylamino)hexyl]-AMPc et le 8-(6-aminohexy])amino-AMPc ; et l'analogue de GMPc est choisi parmi le 8-bromo-GMPc, le dibutyryl-GMPc et le sel de sodium de 8-(4-chlorophénylthio)-guanosine 3',5'-cydique monophosphate.

**3.** Procédé selon la revendication 1 ou 2, comprenant en outre le mélange du sang prélevé avec un agent anticoagulant, de préférence du citrate.

**4.** Procédé selon l'une quelconque des revendications 1 à 3, comprenant en outre le mélange du sang prélevé avec un agent qui favorise l'introduction de l'AMPc ou du composé analogue de celui-ci dans une cellule, dans lequel l'agent est un tensioactif, un solvant ou un réactif qui favorise l'endocytose.

**5.** Procédé selon l'une quelconque des revendications 1 à 4, comprenant en outre le mélange du sang prélevé avec un inhibiteur de cyclooxygénase (COX).

**6.** Procédé selon l'une quelconque des revendications 1 à 5, comprenant en outre le mélange du sang prélevé avec un sang choisi dans le groupe constitué par le glucose, des inhibiteurs de l'élastase granulocytaire, des agents antioxydants, des combinaisons de ceux-ci et des compositions les comprenant.

**7.** Procédé selon l'une quelconque des revendications 1 à 6, dans lequel le sang prélevé après le mélange est

(i) stocké à une température de 10 °C ou moins ; et/ou
(ii) stocké pendant au moins 4 heures.

**8.** Procédé pour effectuer un test de cellules rares dans le sang, le procédé comprenant :

i) l'utilisation d'un sang traité par le procédé selon l'une quelconque des revendications 1 à 7 comme échantillon de sang à soumettre au test ; ou
ii) le traitement du sang prélevé par un procédé selon l'une quelconque des revendications 1 à 7 et la soumission du sang prélevé traité à un test de cellules rares.

**9.** Procédé selon la revendication 8, dans lequel le test de cellules rares comprend un ou plusieurs des éléments suivants :
la détection de cellules rares, la séparation de cellules rares, l'enrichissement de cellules rares, la mesure de cellules rares, l'observation de cellules rares, la récupération de cellules rares, la culture de cellules rares et des tests d'ADN, d'ARN, d'une protéine ou d'éléments similaires contenus dans des cellules rares.

10. Tube de prélèvement sanguin destiné à être utilisé dans un test de cellules rares dans le sang, le tube de prélèvement sanguin comprenant :
de l'AMPc ou un composé analogue de celui-ci choisi dans le groupe constitué par des analogues d'AMPc, de GMPc et d'analogues de GMPc.

11. Tube de prélèvement sanguin selon la revendication 10, dans lequel l'analogue d'AMPc est choisi parmi le sel de sodium de 8-bromo-AMPc, le sel de sodium de 6-monobutyryl-AMPc, le sel de sodium de dibutyryl-AMPc, le sel de sodium de 8-(4-chlorophénylthio)-AMPc, le sel de sodium de 8-(4-chlorophénylthio)-2'-O-méthyl-AMPc, le 2-chloro-8 méthylamino-AMPc, le N-[6-(N-méthylanthraniloylamino)hexyl]-AMPc et le 8-(6-aminohexyl)amino-AMPc ; et l'analogue de GMPc est choisi parmi le 8-bromo-GMPc, le dibutyryl-GMPc et le sel de sodium du 8-(4-chlorophénylthio)-guanosine 3',5'-cydique monophosphate.

12. Tube de prélèvement sanguin selon la revendication 10 ou 11, comprenant en outre un élément choisi dans le groupe constitué par des agents anticoagulants, des inhibiteurs de cyclooxygénase (COX), le glucose, des inhibiteurs d'élastase granulocytaire, des agents antioxydants, des combinaisons de ceux-ci et des compositions les comprenant.

13. Procédé de traitement de sang prélevé, le procédé comprenant :

   (i) le mélange du sang prélevé avec de l'AMPc ou un composé analogue de celui-ci et avec un inhibiteur de cyclooxygénase (COX) ; ou
   (ii) le mélange du sang prélevé avec de l'AMPc et un composé analogue de celui-ci, le composé analogue de l'AMPc étant tel que défini dans la revendication 1 ou 2.

14. Tube de prélèvement sanguin comprenant :

   (i) l'AMPc ou un composé analogue de celui-ci et
   un inhibiteur de cyclooxygénase (COX) ; ou
   (ii) l'AMPc ou un composé analogue de celui-ci,

   dans lequel le composé analogue de l'AMPc est tel que défini dans la revendication 1 ou 2.

15. Utilisation d'un tube de prélèvement sanguin dans un test de cellules rares dans le sang, dans laquelle le tube de prélèvement sanguin est tel que défini dans l'une quelconque des revendications 10 à 12 ou 14.

FIG. 1

Left) DNA-FISH
(fluorescence micrograph: ×100 objective lens)

Right) RNA-FISH
(fluorescence micrograph: ×40 objective lens)

blue (dashed arrow): cell nucleus,
green dot (diamond arrow): ERBB2
red dot (sharp arrow): CEN17

blue (dashed arrow): cell nucleus,
red dot (sharp arrow): PAN-KRT mRNA

FIG. 2A

white circle: cell nucleus,
black dot: ERBB2
white dot: CEN17

white circle: cell nucleus,
white dot: PAN-KRT mRNA

FIG. 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017507328 A
- WO 2013168767 A
- JP 2005501236 A
- JP 2016180753 A

**Non-patent literature cited in the description**

- *CHEMICAL ABSTRACTS, 76939-46-3*
- *CHEMICAL ABSTRACTS, 70253-67-7*
- *CHEMICAL ABSTRACTS, 16980-89-5*
- *CHEMICAL ABSTRACTS, 93882-12-3*
- *CHEMICAL ABSTRACTS, 510774-50-2*
- *CHEMICAL ABSTRACTS, 96990-16-8*
- *CHEMICAL ABSTRACTS, 723313-27-7*
- *CHEMICAL ABSTRACTS, 39824-30-1*
- *CHEMICAL ABSTRACTS, 51116-01-9*
- *CHEMICAL ABSTRACTS, 51116-00-8*
- *CHEMICAL ABSTRACTS, 51239-26-0*
- *CHEMICAL ABSTRACTS, 317-34-0*
- *CHEMICAL ABSTRACTS, 58-08-2*
- *CHEMICAL ABSTRACTS, 69-89-6*
- *CHEMICAL ABSTRACTS, 58-55-9*
- *CHEMICAL ABSTRACTS, 4499-40-5*
- *CHEMICAL ABSTRACTS, 652-37-9*
- *CHEMICAL ABSTRACTS, 10381-75-6*
- *CHEMICAL ABSTRACTS, 83-67-0*
- *CHEMICAL ABSTRACTS, 479-18-5*
- *CHEMICAL ABSTRACTS, 519-37-9*
- *CHEMICAL ABSTRACTS, 73963-72-1*
- *CHEMICAL ABSTRACTS, 78415-72-2*
- *CHEMICAL ABSTRACTS, 60719-84-8*
- *CHEMICAL ABSTRACTS, 74150-27-9*
- *CHEMICAL ABSTRACTS, 823178-43-4*
- *CHEMICAL ABSTRACTS, 68550-75-4*
- *CHEMICAL ABSTRACTS, 77671-31-9*
- *CHEMICAL ABSTRACTS, 78416-81-6*
- *CHEMICAL ABSTRACTS, 37762-06-4*
- *CHEMICAL ABSTRACTS, 171599-83-0*
- *CHEMICAL ABSTRACTS, 224789-15-5*
- *CHEMICAL ABSTRACTS, 171596-29-5*
- *CHEMICAL ABSTRACTS, 78351-75-4*
- *CHEMICAL ABSTRACTS, 330784-47-9*
- *CHEMICAL ABSTRACTS, 268203-93-6*
- *CHEMICAL ABSTRACTS, 334827-98-4*
- *CHEMICAL ABSTRACTS, 227619-96-7*
- *CHEMICAL ABSTRACTS, 58-32-2*
- *CHEMICAL ABSTRACTS, 936449-28-4*
- *CHEMICAL ABSTRACTS, 61413-54-5*
- *CHEMICAL ABSTRACTS, 162401-32-3*
- *CHEMICAL ABSTRACTS, 153259-65-5*
- *CHEMICAL ABSTRACTS, 29925-17-5*
- *CHEMICAL ABSTRACTS, 145739-56-6*
- *CHEMICAL ABSTRACTS, 35838-58-5*
- *CHEMICAL ABSTRACTS, 57076-71-8*
- *CHEMICAL ABSTRACTS, 69975-86-6*
- *CHEMICAL ABSTRACTS, 136145-07-8*
- *CHEMICAL ABSTRACTS, 608141-41-9*
- *CHEMICAL ABSTRACTS, 329306-27-6*
- *CHEMICAL ABSTRACTS, 346629-30-9*
- *CHEMICAL ABSTRACTS, 433695-36-4*
- *CHEMICAL ABSTRACTS, 873541-45-8*
- *CHEMICAL ABSTRACTS, 58-61-7*
- *CHEMICAL ABSTRACTS, 146-77-0*
- *CHEMICAL ABSTRACTS, 88475-69-8*
- *CHEMICAL ABSTRACTS, 35121-78-9*
- *CHEMICAL ABSTRACTS, 55142-85-3*
- *CHEMICAL ABSTRACTS, 113665-84-2*
- *CHEMICAL ABSTRACTS, 66575-29-9*
- *CHEMICAL ABSTRACTS, 138605-00-2*
- *CHEMICAL ABSTRACTS, 54-21-7*
- *CHEMICAL ABSTRACTS, 493-53-8*
- *CHEMICAL ABSTRACTS, 15307-79-6*
- *CHEMICAL ABSTRACTS, 41340-25-4*
- *CHEMICAL ABSTRACTS, 15687-27-1*
- *CHEMICAL ABSTRACTS, 226721-96-6*
- *CHEMICAL ABSTRACTS, 21256-18-8*
- *CHEMICAL ABSTRACTS, 22204-53-1*
- *CHEMICAL ABSTRACTS, 71125-38-7*
- *CHEMICAL ABSTRACTS, 36322-90-4*
- *CHEMICAL ABSTRACTS, 53-86-1*
- *CHEMICAL ABSTRACTS, 188817-13-2*
- *CHEMICAL ABSTRACTS, 38194-50-2*
- *CHEMICAL ABSTRACTS, 501-36-0*
- *CHEMICAL ABSTRACTS, 4394-00-7*
- *CHEMICAL ABSTRACTS, 123653-11-2*
- *CHEMICAL ABSTRACTS, 6385-02-0*
- *CHEMICAL ABSTRACTS, 127373-66-4*
- *CHEMICAL ABSTRACTS, 157341-41-8*
- *CHEMICAL ABSTRACTS, 344930-95-6*
- *CHEMICAL ABSTRACTS, 51798-45-9*
- *CHEMICAL ABSTRACTS, 65144-34-5*
- *CHEMICAL ABSTRACTS, 18719-76-1*
- *CHEMICAL ABSTRACTS, 9035-81-8*

- *CHEMICAL ABSTRACTS,* 51050-59-0
- *CHEMICAL ABSTRACTS,* 198062-54-3
- *CHEMICAL ABSTRACTS,* 675103-36-3
- *CHEMICAL ABSTRACTS,* 1448314-31-5
- *CHEMICAL ABSTRACTS,* 291778-77-3